# EUROPEAN PATENT APPLICATION

(11) **EP 1 661 915 A1**
(43) Date of publication of application: **31.05.2006**
(21) Application number: 04027998.6
(22) Date of filing: 25.11.2004
(51) Int. Cl.: C07K 16/18, A61K 39/395, G01N 33/68, A61P 11/06, A61P 37/08

(54) **Preparation of antibodies against the human chitinase-like protein GL008**

(71) Applicant: GSF-Forschungszentrum für Umwelt und Gesundheit GmbH, 80807 München (DE)
(72) Inventor: Kremmer, Elisabeth, 85354 Freising (DE); Kzhyshkowska, Julia, 68165 Mannheim (DE); Goerdt, Sergij, 68163 Mannheim (DE)
(74) Representative: Reinhard - Skuhra - Weise & Partner

(57) **Abstract**

The present invention is directed to the preparation of antibodies against a human chitinase-like protein. This human chitinase-like protein is considered to be involved in Th2-type inflammation/allergies. Its detection is proposed to be useful for the prognosis/alleviation of effects associated with the glucocorticoid therapy, and for the determination of the individual reactivity of patients on glucocorticoid therapy.

## Description

The present invention is directed to a human chitinase-like protein and its use in diagnosis and therapy. The present invention in particular is directed to a binding agent and inhibitor for this protein and its use in the diagnosis/prognosis and therapy of several diseases, in particular inflammation/allergies, for the prognosis/alleviation of effects associated with the glucocoticoid therapy, cancer and for the determination of the individual reactivity of patients on glucocorticoid therapy.

Rates of asthma, for example in the UK, are currently almost double the EU average, 13.8% of the population compared with 7.2%. In Germany, 4.7% of the population has been diagnosed with the disease, which can prove fatal in the most severe, undiagnosed cases.

In the UK, asthma kills an estimated number of 1,500 people a year and costs the NHS an estimated £850 million to treat. Comparable Asthma rates in other European countries are: Finland: 11.0; Ireland: 10.5; Netherlands: 8.7; EU average: 7.2; France: 6.9; Germany: 4.7.

According to present medical knowledge, asthma is a controllable but not curable disease. The World Health Organisation (WHO) estimates that 100 to 150 million people around the world are asthmatic and the number is growing by 50% every decade. It causes 180,000 deaths a year.

### There are plenty of known triggers for asthma attacks. They include:

Allergens (substances to which some people are allergic) such as pollens, foods, dust, mould, feathers, or animal dander (small scales from animal hair or feathers); irritants in the air such as dirt, cigarette smoke, gases, and odours; Respiratory infections such as colds, flu, sore throats, and bronchitis; too much exertion such as running upstairs too fast or carrying heavy loads (although people with asthma can benefit from a moderate amount of exercise); Emotional stress such as excessive fear or excitement; weather such as very cold air, windy weather, or sudden changes in weather; medication such as acetyl-salicylic acid or related drugs and some drugs used to treat glaucoma and high blood pressure.

Each person with asthma reacts to a different set of triggers. Identifying which triggers apply is important if asthma attacks are to be prevented.

Asthma treatment consists of two main factors - environmental control and medication. Environmental control means simply keeping away from factors - like tobacco smoke or allergens - likely to trigger an attack. Medication is the mainstay of asthma treatment. Because patterns of asthma are different for different people, the specific type of drug treatment varies a lot depending on the frequency, severity, and particular triggers of each patient's episodes.

For people with mild asthma, medication may only be needed before exposure to triggers or when they detect the onset of an attack. Those with more frequent symptoms may take daily medicine as well as using medicine for specific symptoms. In the case of severe, persistent asthma patients may need two or more doses of medicine each day.

The major types of anti-asthma drugs are: corticosteroids, which reduce the inflammation of the airways; anti-allergy drugs, which can be used to prevent an attack, but are of no use after an attack has begun; bronchodilators, of which there are several types. The latter provide temporary relief from asthma symptoms but do not tackle the underlying inflammation.

Systemic corticosteroids (glucocorticoids) are synthetic derivatives of the natural steroid, cortisol, which is produced by the adrenal glands. They are called "systemic" steroids if taken orally or given by injection as opposed to topical corticosteroids, which are applied directly to the skin. They include prednisone, prednisolone, methylprednisolone, betamethasone, dexamethasone, triamcinolone and hydrocortisone.

Systemic steroids work in the same way as natural cortisol, and are prescribed for a large number of serious diseases. Systemic steroids vary in strength. The beneficial effects as well as the side effects are proportional to the dose taken. Steroid dose is commonly characterised into low dose (< 10mg/day of prednisone), medium (10-20 mg/day) and high dose (> 20mg/day). Treatment continuing for more than 3 months is regarded as long term, and results in the majority of undesirable side effects including Cushing's syndrome

Side effects of glucocorticoids are as follows:

Reduction of the own cortisol production; during and after steroid treatment, the adrenal gland produces less of its own cortisol, resulting from hypopituitary-pituitary-adrenal (HPA) axis suppression. For up to twelve months after the steroids are stopped, the lack of steroid response to stress such as infection or trauma could result in severe illness.

Osteoporosis (thinning of the bones) particularly in smokers, postmenopausal women, the elderly, those who are underweight or immobile, and patients with diabetes or lung problems. Osteoporosis is characterized by progressive loss of mineral bone material, resulting in decreasing bone density. Therefore, osteoporosis may result in fractures of the spine, ribs or hip joint with minimal trauma. Such changes in bone structure and density occur after the first year in 10-20% of patients treated with more than 7.5 mg prednisone daily. It is estimated that up to 50% of patients using oral corticosteroids will develop bone fractures. Bone density gives an indication of the risk of fracture due to bone loss. Preventative treatment includes calcium, vitamin D in various forms, oestrogen and bisphosphonates (alendronate, etidronate). Treatment is most effective when started at the same time as the steroids as most bone loss occurs within the first three months. This is most important for people taking more than 7.5 mg of prednisone (or the equivalent dose of another oral corticosteroid) for three months or more.

Further side effects are: reduction in growth in children; muscle weakness, especially of the shoulder muscles and thighs; rarely, avascular necrosis of the femoral head (destruction of the hip joint); diabetes can be precipitated or aggravated; increase in circulating blood fat (triglycerides); redistribution of body fat: moon face, buffalo hump and truncal obesity; salt retention: leg swelling, raised blood pressure, weight increase and heart failure; shakiness and tremor; eye disease, particularly glaucoma (increased intraocular pressure) and posterior subcapsular cataracts; skin thinning resulting in easy bruising, skin tearing after minor injury, slow healing, and stretch marks; acne; psychological effects including insomnia, mood changes, increased energy, excitement, delirium or depression; headaches; increased susceptibility to infections; peptic ulceration; subcutaneous fat atrophy (loss of fat under the skin surface) from injected steroid that does not go deep enough into the muscle.
There are even side effects from reducing the dose; these include tiredness, headaches, muscle and joint aches and depression.

Cataracts are common in patients treated with glucocorticoids. Glaucoma occurs in a significant number of patients and thus all glucocorticoid-treated patients should have their intraocular pressure monitored.

Thus, methods of prediction, minimizing or preventing side effects of glucocorticoid treatment are desired.

According to the prior art, careful evaluation of the patient before and during treatment is important. Patients should be seen at least monthly while on high doses of prednisone, depending on the underlying disease process. A diet low in calories, fat and sodium and high in potassium and calcium is recommended. Alcohol, coffee and nicotine aggravate osteoporosis and peptic ulcers and should be minimized. Exercise is beneficial. Calcium and vitamin D can decrease, but not prevent glucocorticoid-induced osteoporosis. Estrogen treatment can be helpful in postmenopausal women on prednisone in preventing bone loss. Newer medication such as bisphosphonate and calcitonin can prevent bone loss or increase bone density in patients on steroids.

Patients with a family history of heart disease should be followed canefully. All patients should have monthly evaluation of blood pressure and serum glucose and periodic analysis of serum lipids. Any abnormalities should be treated with diet and appropriate medication.

Corticosteroids (glucocorticoids) have been available for treating asthma for more than 25 years, and they are among the most powerful agents at our disposal for managing the disease. But determining their proper role in caring for individual patients can be a difficult clinical problem, since chronic use can lead to serious side effects.

However, there is not a test system available which allows the determination of one's individual susceptibilty to glucocorticoids. Such a system would be highly desirable in order to precisely determine the individual dosage and treatment regimen of a patient in need of a glucocorticoid treatment and to select additional individual blocking components eliminating or decreasing glucocorticoid side effects.

Thus, it is a problem underlying the present invention to provide a new diagnostic and/or therapeutic marker and binding agent therefore, for the diagnosis and/or determination of individual reactivity of patients on glucocorticoid therapy, diagnosis and treatment of inflammation/allergies/asthma, prognosis of glucocorticoid effects and for the diagnosis/prognosis and treatment of cancer. It is a further problem underlying the present invention to provide a method of determining a candidate compound's ability of causing or preventing an allergy, inflammation, asthma or cancer, or for determining glucocorticoid effects, thus making a prognosis and treatment of long term effects of glucocorticoids possible.

These problems are solved by the subject-matter of the independent claims. Preferred embodiments are described in the dependent claims.

The inventors discovered the new putative cytokine GL008 (SEQ ID NO: 1 and 2) which is produced by human macrophages. GL008 belongs to the novel family of Glyco_18 containing chitinase-like proteins. It is a candidate chemokine, for attraction of eosinophils and induction endothelial cells and fibroblast proliferation under dexamethasone treatment during asthma and other allergic diseases. Involvement in asthma of another member of family of chitinase-like proteins with Glyco_18 domain was demonstrated recently (ZHU Z. et al. Acidic mammalian chitinase in asthmatic Th2 inflammation and IL-13 pathway activation (Science, 2004 Jun 11; 304 (5677):1678-82).

As mentioned above, prediction of individual patients reactivity on glucocorticoid therapy is necessary to develop combined therapeutic approaches to eliminate side effects of corticoids. GL008 may be used as a tool for prediction. It is, for example, upregulated in macrophages by dexamethasone, and the level of up-regulation is patient-specific. Thus, for example, the detection of the GL008 level in dexamethasone stimulated human monocytes *in vitro* will allow to examine the individual patients response, predict side effects, and design individual cytokine cocktails for the complex therapy. GL008 belongs to a novel mammalian family of chitinase-like proteins Glyco_18 domain containing proteins. GL008 related family members possess the following physiological activities:
1) chemoattractant for eosinophils and leukocytes (OWHASHI et al., 2000);
2) proliferation of synovial cells and fibroblasts (RECKLIES et al., 2002);
3) adhesion, migration factor, proliferation for vascular cells;
4) inhibition of inflammatory signals (LING and RECKLIES, 2004)

In summary, human chitinase-like Glyco_18 domain containing proteins were found to be associated with following human disorders:
1. Asthma (ZHU et al., 2004);
2. Rheumatoid arthritis and osteoarthritis (KAWASAKI et al., 2001; TSURUHA et al., 2002; STEENBAKKERS et al., 2003; STECK et al., 2002; KNORR 2003);
3. Purulent meningitis (OSTERGAARD, 2002);
4. Ovarian cancer ( DUPONT et al., 2004);
5. Colorectal cancer (CITIN et al., 1999: CITIN et al., 2002);
6. Inflammatory Bowel disease (BERNARDI et al., 2003; VIND et al., 2003);
7. Breast cancer (JENSEN et al., 2003);
8. Liver disorders (SEKINE et al., 2001; TRAN et al., 2000);
9. Glioma (TANWAR et al., 2002).

Glyco_18 containing proteins are also used as diagnostic and prognostic markers for following human disorders:
1. Arthritis (JOHANSEN et al., 1993, JOHANSEN et al., 1999; PELTOMAA 2001; TAKAHASHI et al., 2004);
2. Colorectal cancer (CITIN et al., 1999; CITIN et al., 2002);
3. Ovarain cancer (NOJGAARD et al., 2003; DUPONT et al., 2004);
4. Breast cancer (JOHANSEN et al., 1995; JENSEN et al., 2003);
5. Systemic sclerosis (MONTAGNA et al., 2003);

In contrast to the prior art cited, GL008 was identified as an interacting partner of Stabilin-1 in yeast two hybrid screening. The expression of GL008 mRNA was up-regulated in macrophages under the same stimulation, which is necessary to induce stabilin-1 expression. Once GL008 was found as interacting partner of stabilin-1 in *in vitro* systems, specific antibodies recognizing native and denatured GL008 protein were required 1) to prove that GL008 mRNA synthesis leads to corresponding protein translation (to prove protein existence in human system); 2) to characterise native protein biochemically (size, modifications, processing); 3) to characterize the physiological significance of the GL008/Stabilin-1 complex; 4) to identify the function of the GL008 protein itself.

In the present application, the generation of GL008 immunogen, the design of a test system and the analysis of specificity of novel anti GL008 monoclonal antibodies is disclosed. For example, a GST-fused 50 amino acids fragment of GL008 protein was purified out of *E. coli* in native conditions using affinity chromatography, and used for rat immunization.

Further characterization of monoclonal antibody specificity was performed in 3 steps: 1) Western blotting analysis with immunogen GST-GL008 fragment used as an antigen; 2) Western blotting analysis using 6xHis-tagged full length GL008 as an antigen; 3) Western blotting and indirect immunofluorescence analysis of full length FLAG-tagged GL008 expressed in mammalian cells. Eight monoclonal antibodies specifically recognized GL008 protein in all tests. Western blotting analysis and indirect immunofluorescence in transiently transfected mammalian cells suggested that GL008 undergoes posttranslational processing. Using immunofluorescence analysis and confocal microscopy GL008 protein was shown to localize in large intracellular vesicular structures in CHO-K1 and H1299 cells, transiently or stably transfected with a GL008 expression constructs.

In summary, the inventors obtained novel monoclonal antibodies which can be used for the diagnosis and therapy of several diseases based on the GL008 structure and function.

The following results were *inter alia* obtained:
1. antigen GL008 was identified as a ligand for novel macrophage scavenger receptor Stabilin-1 in yeast two hybrid screen using human placenta cDNA library. The interaction is confirmed by independent *in vitro* binding method (GST-pull down):
2. GL008 is expressed in primary human macrophages isolated from peripheral blood;
3. expression GL008 mRNA and protein level is upregulated by Th2 cytokines (IL4 and TGFb) and glucocorticoids (dexamethasone) in primary and .cultured monocytes and macrophages. The level of cytokines and corticoid effect is donor dependent.

Regarding the above referenced fact that GL008 is belonging to the family of Glyco_18 containing proteins, the following is noted:

GL008 belongs to the human chitinase-like protein family according the domain organization. However, a BLAST search performed by the inventors themselves for a protein similar to already known human chitinase-like proteins was not resulting in the identification of GL008, due to a very low level of similarity. This is indicating that a standard bio-informatic approach is not sufficient for GL008 identification. Remarkably, all other human Glyco_18 domain containing proteins have a very high similarity on the protein level, and can be found as a homologues of YKL-39. YKL39 is the closest homologue of GL008. However, GL008 could not be identified using the bio-informatic approach.

The identity on the protein level is only 20% and is based exclusively on the similarity of Glyco_18 domains.

In contrast thereto, the inventors identified GL008 using functional yeast two-hybrid screening as an interacting partner for the macrophage/endothelial multifunctional receptor stabilin-1. The interaction was confirmed in independent *in vitro* protein binding system. Physiological significance of the finding is supported by the fact, that both proteins are produced by macrophages, and the production is unregulated by the same stimulations.

Comparison of GL008 with other Glyco_18 containing cytokines:
BlastP (protein comparison) GL008 with YKL39: 20% identity
BlastN (nucleotide comparison) GL008 and YKL39: no significant similarity
BlastP (protein comparison) GL008 with YKL40: no significant similarity
BlastN (nucleotide comparison) GL008 and YKL40: no significant similarity
BlastP (protein comparison) YKL39 with YKL40: 51 % identity
BlastN (nucleotide comparison) YKL39 and YKL40: similarity very low

Most of the allergic diseases require glucocorticoid treatment. Glucocorticoid treatment has side effects. An easy *in vitro* assay is required for prediction of individual reactivity of patients to corticoid treatment. The novel antibody of the invention provide the possibility for an easy and sensitive method to test the individual response of patients to corticoids using a small amount of monocytes isolated from peripheral blood samples. Also, ELISA or FACS analysis for easy identification of GL008 in patients' blood is envisioned in the present invention. This method provides the possibility to avoid lung biopsy and to identify directly small changes in GL008 concentration in circulation.

In the prior art, however, there were no methods known to predict individual glucocorticoid reactivity before the patient treatment starts. Thus, there is no possibility to predict *in vitro* the reaction of individual patients before therapy starts; side effects of corticoids are detectable only during the patient treatment, which is a highly undesirable state.

Thus, the present invention for the first time is offering an easy and quick method to test individual response to corticoid treatment before the therapy starts.

As an example, peripheral blood monocytes of a patient will be stimulated with dexamethasone in combination with cytokines *in vitro* for 5 days. The amount of GL008 will be quantified by Western blotting (or ELISA) using rat monclonal anti-GL008 antibodies. The higher the amount of GL008, the higher the individual response to the corticoid treatment will be. In the case of high reactivity, the amount of glucocorticoids will be lowered and/or the precise type of glucocorticoids used will be set accordingly and blocking strategy for the elimination of side effects will be designed individually.

The present invention is in particular directed to the following aspects and embodiments:

According to a first aspect, the present invention provides a binding agent capable of binding to a protein comprising the amino acid sequence of SEQ ID NO: 1 or to a variant of said sequence, wherein said variant comprises one or more insertions, substitutions and/or deletions as compared to the sequence of SEQ ID NO: 1, and wherein the biological activity of the variant is substantially equal to the activity of the protein comprising the unmodified amino acid sequence of SEQ ID NO: 1.

Variants of the protein can also be N-terminal truncations of SEQ ID NO: 1, especially the variant containing the N-terminal amino acid residues 24-73 of SEQ ID NO: 1.

In particular variants of the protein, for example deletions, insertions and/or substitutions in the sequence, which cause for so-called "silent" changes, are considered to be part of the invention. Preferably are such amino acid substitutions the result of substitutions which substitute one amino acid with a similar amino acid with similar structural and/or chemical properties, i.e. conservative amino acid substitutions.

Those amino acid substitutions can be performed on the basis of similarity in polarity, charges, solubility, hydrophobic, hydrophilic, and/or amphipathic (amphiphil) nature of the involved residues. Examples for hydrophobic amino acids are alanine, leucine, isoleucine, valine, proline, phenylalanine, tryptophan and methionine. Polar, neutral amino acids include glycine, serine, threonine, cysteine, thyrosine, asparagine and glutamine. Positively (basic) charged amino acids include arginine, lysine and histidine. And negatively charged amino acids include aspartic acid and glutamic acid.

"Insertions" or "deletions" usually range from one to five amino acids. The allowed degree of variation can be experimentally determined via methodically applied insertions, deletions or substitutions of amino acids in a polypeptide molecule using recombinant DNA methods. The resulting variants can be tested for their biological activity.

According to a preferred embodiment, the invention is directed to a binding agent, which is capable of specifically binding to a sequence corresponding to amino acids 24-73 of SEQ ID NO: 1.

In a second aspect, the present invention provides a binding agent capable of binding to a protein encoded by the nucleic acid sequence of SEQ ID NO: 2 or variants thereof, wherein the variants are each defined as having one or more substitutions, insertions and/or deletions as compared to the sequence of SEQ ID NO: 2, provided that said variants hybridize under moderately stringent conditions to a nucleic acid which comprises the sequence of SEQ ID NO: 2 and further provided that said variants code for a protein with an activity equal to the activity of the protein comprising the unmodified amino acid sequence encoded by SEQ ID NO: 2 or provided that said variants comprise nucleic acid changes due to the degeneracy of the genetic code, which code for the same or a functionally equivalent amino acid as the nucleic acid sequence of SEQ ID NO: 2.

The nucleic acid variants according to the invention also comprise nucleic acid fragments which contain more than 10, preferably more than 15, more than 20, more than 25 or more than 30 and up to 50 nucleotides. The term oligonucleotide includes fragments containing 10 to 50 nucleotides and parts thereof. These sequences can be in any order as long as at least 10 successive nucleotides are according to the invention. These oligonucleotides can be preferably used as primer, for example for RT-PCR or as a probe for in situ hybridization.

According to the state of the art an expert can test which derivatives and possible variations derived from these revealed nucleic acid sequences according to the invention are, are partially or are not appropriate for specific applications like hybridization and PCR assays. The nucleic acid and oligonucleotides of the inventions can also be part of longer DNA or RNA sequences, e.g. flanked by restriction enzyme sites.

Amplification and detection methods are according to the state of the art. The methods are described in detail in protocol books which are known to the expert. Such books are for example Sambrook et al., 1989, Molecular Cloning, A Laboratory Manual, Cold Spring Harbor Press, and all subsequent editions. PCR-methods are described for example in Newton, PCR, BIOS Scientific Publishers Limited, 1994 and all subsequent editions.

As defined above, "variants" are according to the invention especially such nucleic acids, which contain one or more substitutions, insertions and or deletions when compared to the nucleic acids of SEQ ID NO: 2. These lack preferably one, but also 2, 3, 4, or more nucleotides 5' or 3' or within the nucleic acid sequence, or these nucleotides are replaced by others.

The nucleic acid sequences of the present invention comprise also such nucleic acids which contain sequences in essence equivalent to the nucleic acids described in SEQ ID NO: 2. According to the invention nucleic acids can show for example at least about 80%, more typically at least about 90% or 95% sequence identity to the nucleic acids described in SEQ ID NO: 2.

The term "nucleic acid sequence" as used herein generally means a heteropolymer of nucleotides or the sequence of these nucleotides. The term "nucleic acid", as herein used, comprises RNA as well as DNA including cDNA, genomic DNA and synthetic (e.g. chemically synthesized) and to other polymers linked bases such as PNA (peptide nucleic acids).

The invention comprises - as mentioned above ― also such variants which hybridize to the nucleic acids according to the invention at moderately stringent conditions.

Stringent hybridization and wash conditions are in general the reaction conditions for the formation of duplexes between oligonucleotides and the desired target molecules (perfect hybrids) or that only the desired target can be detected. Stringent washing conditions mean 0.2 x SSC (0.03 M NaCl, 0.003 M sodium citrate, pH 7)/0.1% SDS at 65°C. For shorter fragments, e.g. oligonucleotides up to 30 nucleotides, the hybridization temperature is below 65°C, for example at 50°C, preferably above 55°C, but below 65°C. Stringent hybridization temperatures are dependent on the size or length, respectively of the nucleic acid and their nucleic acid composition and will be experimentally determined by the skilled artisan. Moderate stringent hybridization temperatures are for example 42°C und washing conditions with 0.2 x SSC/0.1% SDS at 42°C.

The respective temperature conditions can vary dependent on the chosen experimental conditions and to be tested nucleic acid probe, and have to be adapted appropriately. The detection of the hybridization product can be done for example using X-Ray in the case of radioactive labeled probes or by fluorimetry in the case of fluorescent labeled probes.

The expert can according to the state of the art adapt the chosen procedure, to reach actually moderate stringent conditions and to enable a specific detection method. Appropriate stringent conditions can be determined for example on the basis of reference hybridization. An appropriate nucleic acid or oligonucleotide concentration needs to be used. The hybridization has to occur at an appropriate temperature (the higher the temperature the lower the binding).

Fragments of the nucleic acids according to the invention can be used for example as oligonucleotide primer in detection systems and amplification methods of the GL008 gene and GL008 transcript. The expert can apply these oligonucleotides in state of the art methods. DNA or RNA can be analyzed for the presence of one of the described genes or transcripts applying the appropriate oligonucleotide primers to the to be analyzed probe. The detection of the RNA or DNA of the probe can be achieved for example by PCR methods, which reveal the presence of the specific DNA and/or RNA sequences. All hereinabove described oligonucleotides can also be used as primers, also as primers for reverse transcription of RNA.

The PCR method has the advantage that very small amounts of DNA are detectable. Dependent on the to be analyzed material and the equipment used the temperature conditions and number of cycles of the PCR have to be adjusted. The optimal conditions can be experimentally determined according to standard procedures.

The during the PCR amplification accrued, characteristic, specific DNA fragments can be detected for example by gel electrophoretic or fluorimetric methods with the DNA labeled accordingly. Alternatively, other appropriate, known to the expert, detection systems can be applied.

The DNA or RNA, especially mRNA, of the to be analyzed sample can be an extract or a complex mixture, in which the DNA or RNA to be analyzed are only a very small fraction of the total biological sample. This sample can be analyzed by PCR, e.g. RT-PCR or in hybridization assays. The biological sample can be serum, blood or cells, either isolated or for example as mixture in a tissue. Further, the herein described oligonucleotides can be used for RT-PCR, in situ PCR or in situ hybridization.

In the case of RT-PCR oligonucleotides of the invention are used for PCR amplification of fragments of cDNA matrices, which resulted from the reverse transcription of sample RNA or mRNA. The expression analysis can be qualitative or together with appropriate controls and methods quantitative. For the quantitative analyses an internal standard is used.

The present invention comprises further transcriptional products of the hereinabove described nucleic acids and nucleic acids, which selectively hybridize under moderately stringent conditions to one of these transcriptional products. Preferably this comprises an antisense DNA or RNA in form of a DNA or RNA probe which can hybridize to a transcription product, e.g. mRNA, and can be used in detection systems.

The term "probe" is here defined as a nucleic acid which can bind to a target nucleic acid via one or more kind of chemical binding, usually via complementary base pairing which usually utilizes hydrogen bonds.

For detection the nucleic acids according to the invention are preferably labeled, for example with radioactive labellings, digoxygenin, biotin, peroxidase, fluorescence or alkaline phosphatase. Depending on the label, the detection can be direct or enhanced using indirect immunohistochemistry. Alkaline phosphatase is used as marker enzyme since it develops a sensitive, striking color reaction in the presence of appropriate substrates. Substrates, like p-nitrophenylphosphate, are cleaved and release colored, photometrically measurable products.

In a further embodiment, the present invention provides nucleic acids coupled to a matrix, e.g. nylon membrane, glass or polymers.

In a further embodiment, the present invention includes the invention of a vector (construct) comprising a nucleic acid according to the invention. This vector is preferably an expression vector which contains a nucleic acid according to the invention and one or more regulatory nucleic acid sequences.

Numerous vectors are known to be appropriate for the transformation of bacterial cells, for example plasmids and bacteriophages, like the phage λ, are frequently used as vectors for bacterial hosts. Viral vectors can be used in mammalian and insect cells to express exogenous DNA fragments, e.g. SV 40 and polyoma virus.

The transformation of the host cell can be done alternatively directly using "naked DNA" without the use of a vector.

The protein according to the invention can be produced either in eukaryotic or prokaryotic cells. Examples for eukaryotic cells include mammalian, plant, insect and yeast cells. Appropriate prokaryotic cells include Escherichia coli and Bacillus subtilis.

Preferred mammalian host cells are *CHO, COS, HeLa, 293T, HEH* or *BHK* cells or adult or embryonic stem cells.

In a further embodiment, the present invention includes an antibody or aptamer which recognizes the protein according to the invention.

The antibody is preferably selected from a group, which consists of polyclonal antibodies, monoclonal antibodies, humanized antibodies, chimeric antibodies and synthetic antibodies.

The antibody according to the invention can be additionally linked to a toxic and/or a detectable agent.

The term "antibody", is used herein for intact antibodies as well as antibody fragments, which have a certain ability to selectively bind to an epitop. Such fragments include, without limitations, Fab, F(ab')₂ und Fv antibody fragment. The term "epitop" means any antigen determinant of an antigen, to which the paratop of an antibody can bind. Epitop determinants usually consist of chemically active surface groups of molecules (e.g. amino acid or sugar residues) and usually display a three-dimensional structure as well as specific physical properties.

The antibodies according to the invention can be produced according to any known procedure. For example the pure complete protein according to the invention or a part of it can be produced and used as immunogen, to immunize an animal and to produce specific antibodies.

The production of polyclonal antibodies is commonly known. Detailed protocols can be found for example in Green et al, Production of Polyclonal Antisera, in *Immunochemical Protocols* (Manson, editor), pages 1 - 5 (Humana Press 1992) und Coligan et al, Production of Polyclonal Antisera in Rabbits, Rats, Mice and Hamsters, in *Current Protocols In Immunology,* section 2.4.1 (1992). In addition, the expert is familiar with several techniques regarding the purification and concentration of polyclonal antibodies, as well as of monoclonal antibodies (Coligan et al, Unit 9, *Current Protocols in Immunology,* Wiley Interscience, 1994).

The production of monoclonal antibodies is as well commonly known. Examples include the hybridoma method (Kohler and Milstein, 1975, Nature, 256:495-497, Coligan et al., section 2.5.1 - 2.6.7; and Harlow et al., *Antibodies: A Laboratory Manual,* page 726 (Cold Spring Harbor Pub. 1988).), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

In brief, monoclonal antibodies can be attained by injecting a mixture which contains the protein according to the invention into a rat. The antibody production in the rats is checked via a serum sample. In the case of a sufficient antibody titer, the rat is sacrificed and the spleen is removed to isolate B-cells. The B cells are fused with myeloma cells resulting in hybridomas. The hybridomas are cloned and the clones are analyzed. Positive clones which contain a monoclonal antibody against the protein are selected and the antibodies are isolated from the hybridoma cultures. There are many well established techniques to isolate and purify monoclonal antibodies. Such techniques include affinity chromatography with protein A sepharose, size-exclusion chromatography and ion exchange chromatography. Also see for example, Coligan et al., section 2.7.1 - 2.7.12 and section "Immunglobulin G (IgG)", in *Methods In Molecular Biology,* volume 10, pages 79 - 104 (Humana Press 1992).

According to a still further embodiment, the invention as hereinabove described provides a hybridoma cell line which produces a monoclonal antibody which specifically binds to the protein according to the invention.

According to a preferred embodiment, an inhibitor of the GL008 protein of the invention is provided, wherein said inhibitor is selected from the group consisting of a molecule that reduces the level of mRNA encoding said protein, a molecule that reduces the level of said protein, and a molecule that reduces the biological activity of said protein. The inhibitor may be selected from the group consisting of an antisense nucleic acid, a ribozyme, double stranded RNA, an antibody, a peptide and a peptidomimetic or small molecules which bind to the protein of the present invention. Such an inhibitor may find application for the treatment of several diseases as outlined below by blocking GL008 in its physiological function, for example, as an inductor of cell proliferation.

The invention further includes a pharmaceutical or diagnostic composition comprising a binding agent or an inhibitor of the invention as an active component in combination with a pharmaceutical acceptable carrier and/or diluent and/or auxiliary materials.

In other words, the active components of the present invention are preferably used in such a composition, in doses mixed with an acceptable carrier or carrier material, that the disease can be treated or at least alleviated. Such a composition can (in addition to the active component and the carrier) include filling material, salts, buffer, stabilizers, solubilizers and other materials, which are known state of the art.

The term "pharmaceutical acceptable" material is defined as a non-toxic material, which does not interfere with effectiveness of the biological activity of the active component. The choice of the carrier is dependent on the application.

The pharmaceutical composition can contain additional components which enhance the activity of the active component or which supplement the treatment. Such additional components and/or factors can be part of the pharmaceutical composition to achieve a synergistic effects or to minimize adverse or unwanted effects.

Techniques for the formulation or preparation and application/medication of compounds of the present invention are published in "Remington's Pharmaceutical Sciences", Mack Publishing Co., Easton, PA, latest edition. A therapeutically effective dose of ingredients will be provided in such a composition, which term relates to the amount of a compound which is sufficient to improve the symptoms, for example a treatment, healing, prevention or improvement of such conditions. An appropriate application can include for example oral, dermal, rectal, transmucosal or intestinal application and parenteral application, including intramuscular, subcutaneous, intramedular injections as well as intrathecal, direct intraventricular, intravenous, intraperitoneal or intranasal injections. The intravenous injection is the preferred treatment of a patient.

A typical composition for an intravenous infusion can be produced such that it contains 250 ml sterile Ringer solution and about 10 mg of the antibody of the invention.

An amount which is adequate to reach the therapeutic effect is defined as "therapeutically effective dose". Amounts, which are effective for these applications, depend on the severity of the condition and the general condition of the patient and his immune system. However, the dose range is usually between 0.01 and 100 mg binding agent per dose with a dose of 0.1 to 50 mg and from 1 to 10 mg per patient being preferred. Single or multiple applications after a daily, weekly or monthly treatment regimen can be performed with application rate and samples chosen by the physician in charge.

See also Remington's Pharmaceutical Science (15^{th} edition, Mack Publishing Company, Easton, Ps., 1980).

The active component or mixture of it in the present case can be used for prophylactic and/or therapeutic treatments.

According to a further aspect, the present invention is directed to the use of a binding agent or of an inhibitor or of a composition as defined herein for the treatment of inflammation/allergies, for alleviating effects associated with glucocorticoid therapy or for the treatment of cancer (also for the manufacture of a medicament for achieving same).

Furthermore, in an aspect, the invention provides for the use of a binding agent or a composition as defined hereinabove for the diagnosis and/or determination of individual reactivity of patients on glucocorticoid therapy, diagnosis of inflammation/allergies, prognosis of glucocorticoid effects and for the diagnosis/prognosis of cancer.

According to a preferred embodiment, the present invention provides a method of treating an inflammation, allergy, asthma or cancer or for alleviating the side effects of the glucocorticoid therapy comprising administering an GL008 inhibiting amount of the above defined composition to a patient in need of such treatment.

In a further aspect, an *ex vivo* method for the diagnosis and/or determination of individual reactivity of patients on glucocorticoid therapy, for the diagnosis of inflammation/allergies, prognosis of glucocorticoid effects and for the diagnosis/prognosis of cancer is provided, comprising the steps of:
a) providing a patient sample;
b) qualitative and/or quantitative determination of the amount of protein according to SEQ ID NO: 1, of the nucleic acid according to SEQ ID NO: 2 or translational products thereof in this sample;
c) wherein the amount of protein or nucleic acid as determined in b) being indicative of the reactivity of said patient on glucocorticoid therapy, of inflammation/allergies, of glucocorticoid effects or of cancer.

It is noted that the term "glucocorticoid effects" shall comprise both, wanted (therapeutic) and unwanted side effects.

According to a preferred embodiment, the patient sample contains primary and cultured monocytes and macrophages.

In a further preferred embodiment, the determination in step b) is performed by means of a binding agent as defined herein and/or by means of Western Blotting, RT-PCR, immunohistologic determination, immunoassay, preferably ELISA, MALDI, HPLC, GC, Northern Blotting or a combination thereof.

This test system is useful for the diagnosis/prognosis of a patient's reactivity towards glucocorticoids and, in particular, for the diagnosis of asthma. Thus, a sample will be obtained from the patient and the amount of GL008 mRNA or protein will be qualitatively or quantitatively determined after contacting the sample with a glucocorticoid, an abnormal high amount of the latter compounds being indicative for an abnormally high reactivity towards glucocorticoids.

This test system additionally may be used as a screening method, which is a further embodiment of the invention. For example, the sample is obtained from the patient and the amount of GL008 mRNA or protein will be qualitatively or quantitatively determined after contacting the sample with a glucocorticoid as a reference. In a further step, the same is done, but a candidate compound is added to the system and, again, the amount of GL008 mRNA or protein will be qualitatively or quantitatively determined. If the amount of GL008 is lower than in the reference, the compound will have a capability of reducing the side effects of glucocorticoids *in vivo.*

The same principle may be used to determine, whether a patient is showing an allergy against a certain substance. In this case, the substance is contacted with the sample from the patient in the same manner as with glucocorticoids.

Thus, the present invention is also directed to a method of determining a candidate compound's ability of causing an allergy, asthma, inflammation or cancer, or for determining glucocorticoid effects in a non-human animal, comprising the steps of
administering said candidate compound to said animal,
(b) obtaining a blood or tissue sample from said animal;
(c) qualitative and/or quantitative determination of the amount of protein according to SEQ ID NO: 1, of the nucleic acid according to SEQ ID NO: 2 or translational products thereof, in this sample;
(d) wherein the amount of protein or nucleic acid as determined in (c) being indicative of the candidate compound's ability of causing an allergy, inflammation or cancer or of glucocorticoid effects.

As an example, the above method may be used as a test platform for assessing, whether a specific compound has allergenic potential or not. In this case, the compound in question may be provided to a non-human animal, for example to a mouse or rat, in a first step. In a second step, the amount of GL008 on the mRNA or protein level may be determined, being indicative, whether the compound in question being an allergen or not. Furthermore, this test system may be used for determining, whether a substance has a capability of causing cancer.

According to a further aspect, a method of making an isolated protein as defined hereinabove is provided comprising (a) culturing any one of the above host cells under conditions such that the protein is expressed; and (b) recovering said protein.

Unless otherwise defined, all technical and scientific terms used herein have the same meaning as commonly understood by one of ordinary skill in the art to which this invention pertains. All publications, patent applications, patents, and other references mentioned herein are incorporated by reference in their entirety. In case of conflict, the present specification, including definitions, will control. In addition, the examples are illustrative only and not intended to be limiting.

The invention is now further illustrated by the accompanying drawings, in which:
Fig. 1 is illustrating the domain structure of human Stabilin-1;
Fig. 2 shows the domain organization of GL008;
Fig. 3 is depicting cDNA fragments of GL008;
Fig. 4 is showing PCR products of GL008: GL008-A fragment - 150 bp, GL008-B and GL008-C - 300 bp were amplified with appropriate primers. PCR products were checked by 1.2% agarose gel. 2 µl of the amplified product was loaded onto the gel;
Fig. 5 is illustrating purified PCR products;
Fig. 6 is depicting the verification of pGEX-4T-1-GL008 clones by gel electrophoresis;
Fig. 7 is representing a SDS-PAGE analysis for IPTG Gst-GL008 stimulation;
Fig. 8 is showing a SDS-PAGE analysis of protein purification;
Fig. 9 is showing a SDS-PAGE analysis of GST-GL008-A fractions;
Fig. 10 is showing SDS-PAGE analysis of purified Gst-GL008-A protein;
Fig. 11 is showing the verification of pET28a-GL008 clones by electrophoresis;
Fig. 12 is showing the SDS-PAGE analysis of 6xHis-GL008 expression in *E.coli*;
Fig. 13 is showing the Western blot analysis of 6xHis-GL008 full length purification;
Fig. 14 is showing the Western blot analysis with anti-FLAG Ab;
Fig. 15 is an immunofluorescence with anti-FLAG antibody;
Fig. 16 is showing the Western blot analysis for hybridoma supernatants 1 - 10;
Fig. 17 is showing the Western blot analysis for hybridoma supernatants 11 - 20;
Fig. 18 is showing the Western blot analysis for hybridoma supernatants 21 - 28;
Fig. 19 is showing the Western blot analysis of hybridoma supernatants with His-GL008;
Fig 20 is showing the Western blot analysis of hybridoma supernatants with transfected cells;
Fig. 21 is showing the immunofluorescence in transfected CHO cells;
Fig. 22 Real-time PCR analysis of GL008 mRNA expression in human primary macrophages under different stimulations;
Fig. 23 Western blotting analysis of GL008 protein expression in human primary macrophages under different stimulations.

### Examples:

### Stabilin-1: identification, histology and cloning

One of highly specific markers of alternatively activated macrophages is stabilin-1. Originally stabilin-1 was identified as an MS-1 antigen expressed on non-continuous and angiogenic endothelial cells and by alternatively activated macrophages (24). Mouse mAb MS-1, raised against human spleen, detected an endothelial cell antigen abundantly expressed by the sinusoidal endothelia of spleen, lymph node, liver, and adrenal cortex, but absent from nonsinusoidal continuous endothelia in these organs. Immunoelectron microscopy of splenic tissue demonstrates that the MS-1 antigen is predominantly deposited at zones of intercellular contact between adjacent sinusoidal endothelial cells. mAb MS-1 immunoprecipitates a major protein of 215 kD and minor proteins of 320 and 120 kD from splenic extracts as analyzed by SDS-PAGE with reduction. Extensive immunohistochemical studies revealed that MS-1 antigen can also be expressed by endothelia of continuous origin under certain pathological conditions. In skin lesions, MS-1 antigen expression by endothelial cells of continuous origin is frequently observed in wound healing tissue, in cutaneous T-cell lymphoma, in psoriasis, and in melanoma metastasis (24;26).

The MS-1 antigen was isolated by immunoprecipitation from primary human alternatively activated macrophages after stimulation with IL-4 and dexamathasone. The precipitated MS-1 antigen was purified by affinity chromatography using MS-1 antibody and the peptides were analyzed by matrix assisted laser-desorption-ionization time of flight MS (MALDI-TOF-MS) (27). The cloning of cDNA for human stabilin-1 (hStabilin-1) was based on the MALDI-TOF MS analysis of the purified MS-1 antigen. Human stabilin-1 clone D87433 was identified after the evaluation of MALDI-TOF MS chromatogram with the MS Fit and PepIdent programs. The total length of the D87433 clone was 6777 bp. A polyclonal antibody made against recombinant human stabilin-1 confirmed the expression of stabilin-1 protein in splenic sinus endothelial cells *in vivo* and in alternatively activated macrophages *in vitro* (27).

### Structure of stabilin-1

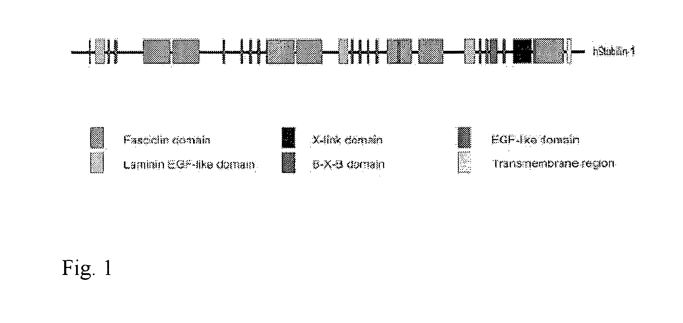

The analysis of the primary structure of hstabilin-1 revealed a protein with an apparent molecular mass of 275 kDa deduced from the amino acid sequence. Stabilin-1 protein shows a mixed content of α-helical and β-sheet secondary structures according to PREDATOR analysis. Scanning the protein sequence using the PROSITE and Pfam databases showed that stabilin-1 is a type-1 transmembrane protein, containing large extracellular part, transmembrane domain and short cytoplasmic tail. Extracellular part contains 7 fasciclin domains and multiple epidermal growth factors (EGF) like domains (Fig. 1). In addition, there is one X-link domain common to hyaluronan (HA) binding proteins (27). Figure 1: Domain structure of human Stabilin-1 (27). This domain organization suggests involvement of stabilin-1 in numerous protein-protein interactions.

Functional analysis revealed, that stabilin-1 is involved in two cellular processes: endocytosis of extracellular ligands and receptor-mediated sorting of newly synthesized proteins forwarding them for secretion.

Using flow cytometry, stabilin-1 was shown to be expressed on the surface of interleukin-4/dexamethasone-stimulated macrophages. By immunofluorescense and confocal microscopy we established, that stabilin-1 is preferentially localised in EEA1-positive early/ sorting endosomes and in recycling endosomes identified by transferrin endocytosis. Association of stabilin-1 was infrequently seen with p62 lck ligand-positive late endosomes and with CD63- positive lysosomes; but Stabilin-1 was not found in Lamp-1-positive lysosomes. Stabilin-1 was also found in trans-Golgi network, but not in Golgi-stack structures. In vitro studies revealed, that the cytoplasmic tail of stabilin-1, but not of stabilin-2 directly interacts with GGAs - recently discovered adaptors mediating traffic between Golgi and endosomal/lysosomal compartment. Binding to Stabilin-1 in GST pull-down assay was shown for GGA1, GGA2, and GGA3L but not GGA3S. The association was confirmed by demonstration of co-localisation of stabilin-1 with GGA2 and GGA3 in trans-Golgi network of human macrophages. This co-localisation was disrupted by brefeldin treatment and resulted in concentration of stabilin-1 in perinuclear areas (Kzhyshkowska, in press).

GL008 protein is the first ligand which is identified as binding partner for human stabilin-1 in several in vitro systems.

### Identification of GL008 as binding partner of stabilin-1

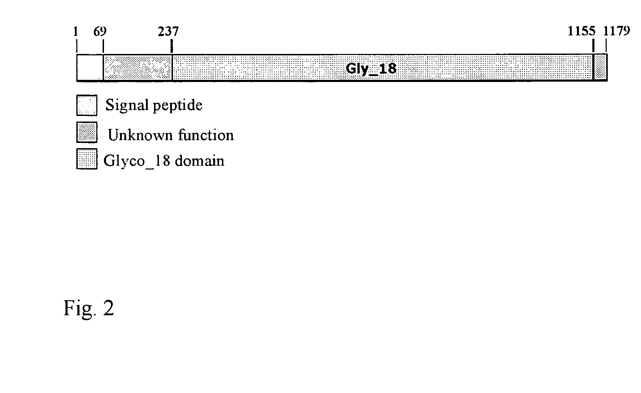

To analyze molecular mechanism of stabilin-1 functioning in alternatively activated macrophages and to identify interacting proteins, yeast two hybrid screening was performed. Since stabilin-1 positive macrophages are found in high amount in placenta human placental cDNA library was screened with stabilin-1 fragment as a bait. The fragment contained extracellular part including epidermal growth factor (EGF) like domain fused to fasciclin domain 7, transmembrane region and intracellular C-terminal fragment with presumed sorting/signaling functions. Proteins found to interact with stabilin-1 in two hybrid system belong to tree major groups: extracellular ligands, components of intracellular trafficking machinery, and secreted cargo-ligands. A novel GL008 protein belongs to the last group. Bioinformatic analysis revealed that GL008 contains conservative Glyco_18 domain and therefore belongs to the glycosyl hydrolase family 18 (Figure 2). Glyco_18 domain is present in proteins comprising hydrolase family 18; these proteins are found in a wide range of organisms, including viruses (28), protozoan parasites (29), bacteria (30), fungi (31), plants (32), insects (33), and mammals. Glyco_18 containing proteins have been found to possess a common (α /β)₈ - barrel domain, consisting of eight α helices and eight β-strands (34-36).

There are several know mammalian protein containing this domain. According to their similarity to known bacterial and plant chitinases, mammalian proteins containing Glyco_18 domain are named chitinase-like proteins, although no chitinolytic activity for these mammalian protein was demonstrated. Mammalian chitinase like protein were shown to bind different sugar structure in *in vitro* assay, possess hydrolytic and transglycosylation activity, and to be involved in chemotaxis of eosinophils *in vivo* (37). Several chitinase-like proteins were demonstrated to be secreted by macrophages (38;39). Analysis of GL008 mRNA expression revealed, that GL008 is expressed in macrophages, and its expression is up-regulated under stimulation with IL-4 and dexamethasone (Kzhyshkowska, unpublished). Figure 2: Domain organization of GL008.

### Sequence of GL008

The nucleotide and deduced amino acid sequence of GL008 are available in National Center for Biotechnology Information (NCBI) GenBank database under accession number BC000001. The cDNA sequence of GL008 (1481 bp) (SEQ ID NO:2 is starting with ATG and ending with TAG):

1 ggcgtttgtg gccgtccggc tccctgacat gcagatttcc acccagaaga cagagaagga
61 gccagtggtc atggaatggg ctggggtcaa agactgggtg cctgggagct gaggcagcca
121 ccgtttcagc ctggccagcc ctctggaccc cgaggttgga ccctactgtg acacacctac
181 catgcggaca ctcttcaacc tcctctggct tgccctggcc tgcagccctg ttcacactac
241 cctgtcaaag tcagatgcca aaaaagccgc ctcaaagacg ctgctggaga agagtcagtt
301 ttcagataag ccggtgcaag accggggttt ggtggtgacg gacctcaaag ctgagagtgt
361 ggttcttgag catcgcagct actgctcggc aaaggcccgg gacagacact ttgctgggga
421 tgtactgggc tatgtcactc catggaacag ccatggctac gatgtcacca aggtctttgg
481 gagcaagttc acacagatct cacccgtctg gctgcagctg aagagacgtg gccgtgagat
541 gtttgaggtc acgggcctcc acgacgtgga ccaagggtgg atgcgagctg tcaggaagca
601 tgccaagggc ctgcacatag tgcctcggct cctgtttgag gactggactt acgatgattt
661 ccggaacgtc ttagacagtg aggatgagat agaggagctg agcaagaccg tggtccaggt
721 ggcaaagaac cagcatttcg atggcttcgt ggtggaggtc tggaaccagc tgctaagcca
781 gaagcgcgtg ggcctcatcc acatgctcac ccacttggcc gaggctctgc accaggcccg

841 gctgctggcc ctcctggtca tcccgcctgc catcaccccc gggaccgacc agctgggcat
901 gttcacgcac aaggagtttg agcagctggc ccccgtgctg gatggtttca gcctcatgac
961 ctacgactac tctacagcgc atcagcctgg ccctaatgca cccctgtcct gggttcgagc
1021 ctgcgtccag gtcctggacc cgaagtccaa gtggcgaagc aaaatcctcc tggggctcaa
1081 cttctatggt atggactacg cgacctccaa ggatgcccgt gagcctgttg tcggggccag
1141 gtacatccag acactgaagg accacaggcc ccggatggtg tgggacagcc aggtctcaga
1201 gcacttcttc gagtacaaga agagccgcag tgggaggcac gtcgtcttct acccaaccct
1261 gaagtccctg caggtgcggc tggagctggc ccgggagctg ggcgttgggg tctctatctg
1321 ggagctgggc cagggcctgg actacttcta cgacctgctc taggtgggca ttgcggcctc
1381 cgcggtggac gtgttctttt ctaagccatg gagtgagtga gcaggtgtga aatacaggcc
1441 tccactccgt ttgctgtgaa aaaaaaaaaa aaaaaaaaaa a

The isolated coding sequence (SEQ ID NO: 2) is the following:

atggaatggg ctggggtcaa agactgggtg cctgggagct gaggcagcca
ccgtttcagc ctggccagcc ctctggaccc cgaggttgga ccctactgtg acacacctac
catgcggaca ctcttcaacc tcctctggct tgccctggcc tgcagccctg ttcacactac
cctgtcaaag tcagatgcca aaaaagccgc ctcaaagacg ctgctggaga agagtcagtt
ttcagataag ccggtgcaag accggggttt ggtggtgacg gacctcaaag ctgagagtgt
ggttcttgag catcgcagct actgctcggc aaaggcccgg gacagacact ttgctgggga
tgtactgggc tatgtcactc catggaacag ccatggctac gatgtcacca aggtctttgg
gagcaagttc acacagatct cacccgtctg gctgcagctg aagagacgtg gccgtgagat
gtttgaggtc acgggcctcc acgacgtgga ccaagggtgg atgcgagctg tcaggaagca
tgccaagggc ctgcacatag tgcctcggct cctgtttgag gactggactt acgatgattt
ccggaacgtc ttagacagtg aggatgagat agaggagctg agcaagaccg tggtccaggt
ggcaaagaac cagcatttcg atggcttcgt ggtggaggtc tggaaccagc tgctaagcca
gaagcgcgtg ggcctcatcc acatgctcac ccacttggcc gaggctctgc accaggcccg
gctgctggcc ctcctggtca tcccgcctgc catcaccccc gggaccgacc agctgggcat
gttcacgcac aaggagtttg agcagctggc ccccgtgctg gatggtttca gcctcatgac
ctacgactac tctacagcgc atcagcctgg ccctaatgca cccctgtcct gggttcgagc
ctgcgtccag gtcctggacc cgaagtccaa gtggcgaagc aaaatcctcc tggggctcaa
cttctatggt atggactacg cgacctccaa ggatgcccgt gagcctgttg tcggggccag
gtacatccag acactgaagg accacaggcc ccggatggtg tgggacagcc aggtctcaga
gcacttcttc gagtacaaga agagccgcag tgggaggcac gtcgtcttct acccaaccct
gaagtccctg caggtgcggc tggagctggc ccgggagctg ggcgttgggg tctctatctg

ggagctgggc cagggcctgg actacttcta cgacctgctc tag

The deduced amino acid sequence of GL008 is as follows (393 aa; SEQ ID NO:1)):

1 
61 
121 
181 
241 
301 
361 

### GL008 homologues in other species

Proteins homologues to human GL008 are found in bacteria, plants, insects and other mammals, indicating that GL008 possess highly conservative function, however neither of GL008 similar protein in other species was described in literature up to date.

**Table 1. Glyco_18 containing proteins with similarity to human GL008.**

| | Full length GL008 | | Glyco_18 domain | |
|---|---|---|---|---|
| | Accession | Identity | Accession | Identity |
| Mouse | AAH06876 | 85% | AAH06876 | 91 % |
| | AAH61063 | 85% | AAH61063 | 91 % |
| | BAB29142 | 83 % | BAB29142 | 92 % |
| | NP_080798 | 80 % | NP_080798 | 91 % |
| | BAC34365 | 80 % | BAC34365 | 91 % |
| Rat | AAP92643 | 70 % | AAP92643 | 72 % |
| | XP_215122 | 55 % | XP_215122 | 52 % |
| | XP_347181 | 55 % | XP_347181 | 52 % |
| Drosophila | NP 609190 | 41 % | NP 609190 | 41 % |
| | AAF52614 | 41 % | AAF52614 | 41 % |
| | AAL28794 | 41 % | AAL28794 | 41 % |
| Arabidopsis | NP_192013 | 35 % | NP_192013 | 36 % |
| | G85013 | 35 % | G85013 | 36 % |
| | CAB80913 | 35 % | CAB80913 | 36 % |
| | T01733 | 27 % | T01733 | 30 % |
| | AAB61039 | 27 % | AAB61039 | 30 % |
| | AAM91562 | 30 % | AAM91562 | 33 % |
| Oryza | NP_917720 | 35 % | NP_917720 | 34 % |
| | BAB67973 | 35 % | BAB67973 | 34 % |
| | BAB 17173 | 31 % | BAB 17173 | 36 % |
| Bacteria | CAE63592 | 28 % | CAE63592 | 28 % |
| | CAE62469 | 27 % | CAE62469 | 26 % |
| | D88949 | 23 % | D88949 | 22 % |
| | AAD34651 | 23 % | AAD34651 | 22 % |
| | ZP_00097013 | 24 % | ZP_0009701 | 25 % |
| | NP_391282 | 18 % | NP_391282 | 18 % |
| | H70030 | 18 % | H70030 | 18 % |
| | P37531 | 24 % | NP_348183 | 24 % |
| | NP 387897 | 24% | H97091 | 24 % |
| | ZP_00098589 | 23 % | NP 623337 | 23 % |
| | NP_348183 | 27 % | NP_348183 | 27 % |
| | H97091 | 27 % | H97091 | 27 % |

### Mammalian proteins containing Glyco_18 domain

Several mammalian proteins containing Glyco_18 domain were recently described and can be divided into 5 major groups according to their similarity, domain organisation and catalytic activity. Presence of chitin-binding domain and ability to use chitin as a substrate indicate that chitiotriosidase and AMCase can be considered as true chitinases. Other Glyco_18-containing proteins do not contain standard chitin binding domain, but bind chitin or other sugars, and can be named chitinase-like proteins. They are involved in wide spectrum of physiological processes including chemotactic activity for eosinophils and other lymphoid cells (37), induction of cell proliferation, adhesion and migration; and fertilisation mechanism (40-42). Identified by us GL008 has no significant homology to other mammalian Glyco_18 containing proteins (max 20% homology to YKL-39), and therefore constitute the sixth group.

Until recently, it has been generally stated that mammals lack chitinases. However, the identification of chitiotriosidase and AMCase has revealed the presence of chitin degrading enzyme in human and mouse (38;43). These enzymes exhibit homology to conservative chitinases found in viruses, protozoan parasites, plants, bacteria, fungi and insects (32;44). Since, chitin is an integral component of fungal cell wall; a defensive function has been suggested for the mammalian chitinases enzyme (45). The elevated levels Glyco_18 containing proteins is associated in humans with lysosomal storage disorder (46), arthritis (47;48), glioblastomas (49) and Streptococcus pneumoniae bacteremia (50), but physiological role of these secreted proteins as well as molecular mechanism of their action remains to be elucidated. Identification, functional characteristics, biochemical properties and correlation with pathologies of selected best investigated mammalian Glyco_18 containing proteins are described below.

All Glyco_18 containing mammalian proteins are involved in multiple disorders. As anexample, the eosinophil chemotactic cytokine (ECF-L)/Ym1 is described below

Several research groups identified independently murine chitinase-like protein containing Glyco_18 domain but lacking chitin-binding domain. Owhashi et al identified a novel eosinophil chemotactic cytokine (ECF-L) in culture supernatant of splenocytes of S. *japonicum*-infected mice (37). High level of ECF-L mRNA expression was detected in spleen, bone marrow, lung and heart. ECF-L possess eosinophil chemotactic activity *in vivo* and in addition was able to attract T lymphocytes and bone marrow polymorphonuclear *leukocytes in vitro,* and this activity can be dependent on the presence of CXC motif in N-terminal part of the protein. ECF-L is not expected to exhibit hydrolytic activity since the replacement of conservative aminoacids in Glyco _18 domain.

Ym1, identical to ECF-L, was found to be transiently expressed in mice infected with *Trichinella spiralis* and described as novel macrophage lectin (56). Ym1 was shown to be a single chain polypeptide (45 kDa) with a strong tendency to crystallize at its isoelectric point (pI 5,7) . Ym1 binds to saccharides with free amine group, such as GlcN, GalN, or GlcN polymers, and the binding activity is enhanced more than 1000-fold due to the clustering effect. Specific binding of Ym1 to heparin suggests that heparin/heparan sulfate may be its physiological ligand in vivo during inflammation and/or tissue remodeling.

Harbord et al discovered crystallized Ym1 in aged lung and chronic inflammation in mouse model of chronic granulomatous disease (57). Ym1 was found in organs of the lymphoreticular system, in lung and distal stomach. Ym1 was shown to be a neutrophil granule protein with weak β-N-acetylglucosaminidase activity. Formation of Ym1 crystals was a consequence of excessive Ym1 release during acute inflammation.

Physiological function of Ym1 remains unclear; Ym1 is believed to play important role in digestion of bacteria and fungi during acute inflammation. Ym1 was also suggested to be associate with Th2-based immune response in mice (58).

### Antigen preparation

### Cloning of GL008 fragments cDNA fragments of GL008 for cloning

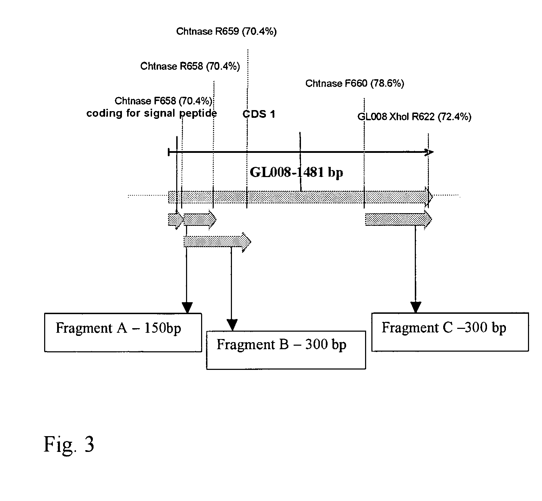

Since previous results demonstrated that the full-length GL008 protein is packed in inclusion bodies when expressed in *E.coli,* three different cDNA fragments were tested to be suitable for purification under native conditions. Two fragments GL008-A and GL008-B coding for 50 and 100 N-terminal amino acids respectively and GL008-C coding for 100 C-terminal amino acids were selected for cloning into pGEX-4T-1 vector down stream to glutathione-S-transferase (GST) gene to generate GST fused protein fragments. Fig. 3: cDNA fragments of GL008.

### PCR amplification of GL008 fragments

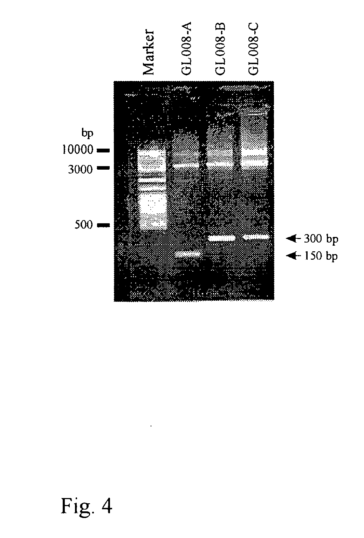

cDNA fragments of GL008-A, -B and -C were amplified by PCR using primers listed in Table 2. Primers were designed with EcoRI recognition sequence on 5' end of the forward primer and XhoI recognition sequence on 5' end of the reverse primer to produce a PCR product, containing EcoRI and XhoI restriction sites on 5' and 3' ends respectively. Restriction sites were introduced in the positions allowing the fusion of the opened reading frame (ORF) of GL008 fragment with the ORF of GST. Amplified products were controlled by performing agarose gel electrophoresis (Fig. 4).

**Table 2: Primers for PCR**

| **cDNA Fragment** | **Primers** |
|---|---|
| GL008-A | Chtnase F658 / R658 |
| GL008-B | Chtnase F658 / R659 |
| GL008-C | Chtnase F660 / R622 |

Fig.4: PCR products of GL008: GL008-A fragment - 150 bp, GL008-B and GL008-C - 300 bp were amplified with appropriate primers. PCR products were checked by 1.2% agarose gel. 2 µl of the amplified product was loaded onto the gel.

### Purification of PCR products

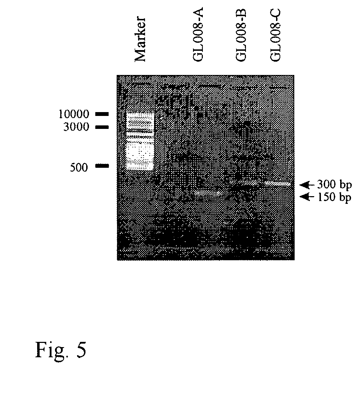

PCR products were purified with GFX DNA - gel band purification kit prior to further use to remove primers, free nucleotides and to exchange the buffers. DNA was eluted from the column with 50 µl of distilled water. The amount of purified DNA was estimated by performing agarose gel electrophoresis (Fig.5). Fig. 5: Purified PCR products.

### Insert and vector preparation

PCR products of GL008 fragments (A, B and C) and pGEX-4T-1 vector were prepared by digesting them with EcoRI and XhoI. Vector was dephosphorylated with CIAP. The total amount of digested vector DNA and PCR products were separated in agarose gels and purified using GFX kit. The resulting purified DNA was used for ligation.

### Ligation and transformation

To generate final construct, digested GL008 fragments (A, B and C) and pGEX-4T-1 vector were ligated using rapid ligation kit. Ligation mix was transformed into *E.coli* DH5α. The efficiency of specific ligation was controlled by parallel ligation of the vector without addition of the insert. The ligation efficiency was assessed by the colony number on the corresponding plates. Typical ligation efficiency was 10: 1 - 20:1 (vector/insert ligation vs. vector control ligation). To select correct clones, mini preparations of plasmid DNA were performed.

### MiniprepDNA analysis

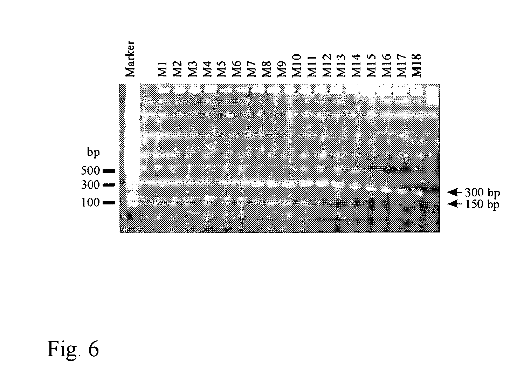

Six independent clones were analyzed for each construct using miniprep protocol. Clones were checked for the correct insert by digesting the isolated plasmid DNA with EcoRI and XhoI. Digests were loaded onto the 1.2 % agarose gels and electrophoresis was carried out. Except miniprep M 18, all other plasmid DNA contained respective GL008 fragment (Fig. 6). Plasmid DNA M1, M7 and M13 containing respective GL008- A, B and C fragments were retransformation in production strain - BL21 (DE3) RIL for the expression and purification of GL008 protein fragment. Fig. 6: Verification of pGEX-4T-1-GL008 clones by gel electrophoresis.

M1-M6 - minipreps of pGEX-4T-1-GL008-A, M7-M12 - minipreps of pGEX-4T-1-GL008-B, M13-M18 - minipreps of pGEX-4T-1-GL008-C.
Small scale expression of GST-fused GL008 fragments

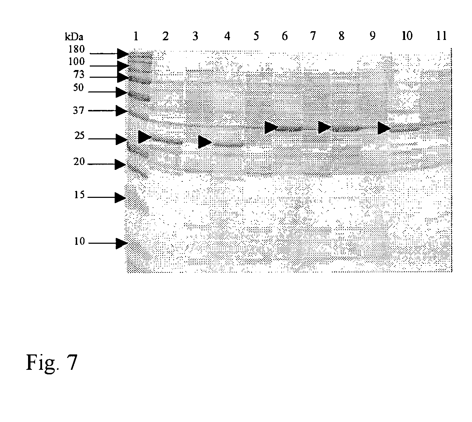

IPTG (isopropyl thiogalactoside) is an artificial inducer of the Lac operon. GL008 fragments were cloned into pGEX 4T-1 vector under the control of IPTG inducible *tac* promoter and internal *lac*I^{q} gene. LacI^{q} gene product is a repressor protein that binds to the operator region of the *tac* promoter preventing the expression until induction by IPTG. Bacterial clones obtained after transformation containing BL21-pGEX-4T-1-GST-GL008-A, BL21-pGEX-4T-1-GST-GL008-B, and BL21-pGEX-4T-1-GST-GL008-C were grown overnight in 5ml LB/Amp medium and at 37°C, 250 rpm. The next day, aliquot of overnight culture was inoculated in 7 ml LB/Amp medium to produce the starting OD₆₀₀ in the range 0.1 - 0.15. Then the culture was grown until OD₆₀₀ reached 0.5 - 0.6. Bacteria were stimulated by IPTG for 3 hours. The stimulation efficiency was controlled by comparing stimulated *E.coli* with non-stimulated ones by performing SDS-PAGE and subsequent staining of the gel (Fig. 7).

Fig 7: SDS-PAGE analysis for IPTG Gst-GL008 stimulation:
Lane 1 - Marker
Lanes 2, 4, 6, 8, and 10 - stimulated with IPTG
Lanes 3, 5, 7, 9, and 11 -non-stimulated
Lanes 2 and 3 - Gst-GL008-A mini-1
Lanes 4 and 5 - Gst-GL008-A mini-2
Lanes 6 and 7 - Gst-G1008-B mini-1
Lanes 8 and 9 - Gst-G1008-B mini-2
Lanes 10 and 11 - Gst-GL008-C mini-1

The protein bands of approximately 30 kDa in lanes 2 and 4 (marked) correspond to the stimulated GST-GL008-A protein, and the protein bands of approximately 37 kDa in lanes 6, 8, and 10 (marked) correspond to the stimulated GST-GL008-B and GST-GL008-C protein respectively (Fig.7).

### Protein purification

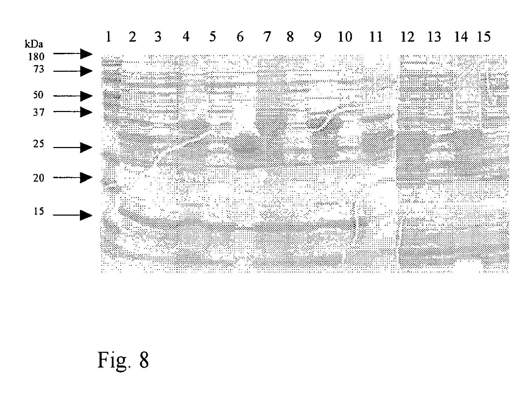

IPTG stimulated bacterial cultures of GST-GL008-A, GST-GL008-B and GST-GL008-C were purified by affinity chromatography using glutathione sepharose beads. Protein samples from each step of purification were checked by SDS-PAGE. This revealed that, GST-GL008-B and GST-GL008-C protein fragments remained in the non-soluble fraction after lysis and ultrasonic homogenization (lane 9 and 14 respectively in Fig. 8). However GST-GL008-A fragment was efficiently solubilized under native conditions (Lane 6 in Fig. 8). Fig.8: SDS-PAGE analysis of protein purification.
Lane 1 - Marker
Lanes 2-6 - GST-GL008-A purification samples
Lanes 7-11 - GST-GL008-B purification samples
Lanes 12-15 - GST-GL008-C purification samples
Lanes 2, 7, and 12 - after sonification step
Lanes 3, 8 and 13 - cell lysates after centrifugation step
Lanes 4, 9 and 14 - Pellet after centrifugation step
Lanes 5 and 10 - supernatant after incubation with glutathione beads
Lanes 6, 11 and 15 - protein bound to glutathione sepharose beads

### Preparative purification of GST-GL008-A protein

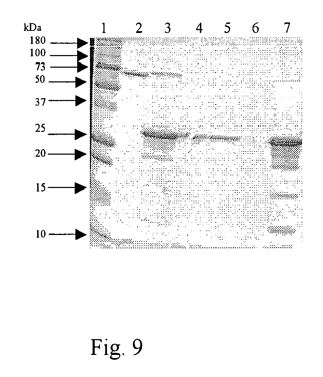

BL21 bacterial clone transformed with pGEX-4T-1-GST-GL008-A was grown overnight in 100 ml LB/amp medium and at 37°C, 250 rpm. On the next day, 500 ml of LB/amp medium was inoculated with overnight culture. Then the culture was grown until OD reached to 0.5 - 0.6. Culture was then stimulated by IPTG for 3 hours. Protein purification from stimulated bacteria was done. GST-GL008-A protein was eluted from the protein bound glutathione sepharose beads and four elution fractions (F1, F2, F3 and F4) 500 µl each were collected. Except fraction F4, all other fractions contained purified GST-GL008-A protein (Fig. 9).

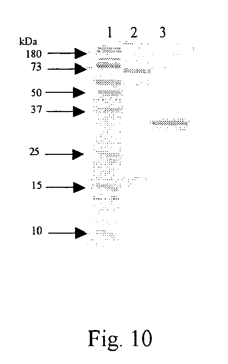

Fig.9: SDS-PAGE analysis of GST-GL008-A fractions
Lane 1: Marker
Lane 2: 2 µg BSA standard
Lanes 3 to 6: - GST-GL008-A elution fractions F1 to F4 respectively
Lane 7: GST-GL008-A bound to glutathione sepharose beads
   Fractions F1 and F2 were pooled and dialyzed against 5 liters 1x PBS overnight. The dialyzed protein was concentrated using Vivaspin concentrator to the final concentration of 1 µg/µl. Obtained protein purity was checked on SDS-PAGE (Fig. 10).
   Fig 10: SDS-PAGE analysis of purified Gst-GL008-A protein:
   Lane 1: Marker; lane 2: 2 µg BSA standard; lane 3: 3 µg purified GST-GL008-A protein.

### Monoclonal anti-GL008 antibody production

Immunization, fusion and primary screening were performed by Dr. E. Krammer, GSF-Forschungszentrum, Institut für Molecular Immunologie, München. Three months after antigen delivery, we received 28 hybridoma supernatants of independent clones, preliminary analyzed in ELISA with GST and GST-GL008-A. Delivered supernatants recognized GST-GL008-A, but not GST alone.

### Generation of the immunogen - positive experience

Previous observation indicated that expression of full length recombinant GST-fused GL008 resulted in the formation of inclusion bodies in bacteria and the protein was insoluble under native conditions. This was in consistence with published observations made for other chitinase-like proteins that formed crystals in mammalian cells (52). To overcome this problem and to generate soluble immunogen, three GST-fused GL008 fragments were tested for their expression efficiency and solubility of the recombinant protein under native conditions. Following fragments were selected for expression: GL008-A containing 50 amino acids and GL008-B containing 100 amino acids from the N-terminal part of GL008; and GL008-C containing 100 amino acids from the C-terminal part of GL008. All three cDNA fragments were cloned in pGEX-4T-1 vector as GST-fusion proteins. GST-tag stabilizes the recombinant immunogen during the immunization procedure. All three recombinant proteins were expressed in *E.coli* on a high level, however only the GL008-A fragment was well soluble under native conditions. GST-GL008-A was successfully purified by affinity chromatography on glutathione sepharose beads and efficiently eluted in the presence of free glutathione. Rats were immunized with the purified antigen according to the standard protocol and the first screening round revealed that 28 hybridomas recognize immunogen, but not GST in ELISA. To test the specificity of these 28 hybridomas we designed a multi-step test system.

### Preparation of test system for anti-GL008 antibody screening

During the immunization period, the test system for the analysis of the new antibodies was designed. The aim was to analyze the specificity of obtained monoclonal antibodies in Western blotting and immunofluorescence with the protein purified out of *E.coli* and/or the protein expressed in mammalian cells.

Test system contained:
Purified from *E.coli* GST-GL008-A immunogen for Western blotting.
Purified from *E.coli* 6xHis-tagged full length GL008 for Western blotting;
Transiently transfected mammalian cells expressing FLAG-tagged full length GL008 protein for Western blotting and immunofluorescence.
6xHis-GL008 full length construct cloning, expression and protein purification
To test the supernatants containing monoclonal anti-GL008 antibody, full length GL008 protein with His-tag was purified.
Cloning of 6xHis-GL008 full length

Full length GL008 cDNA was cloned into pET28a+ vector in the frame with 6xHis tag sequence. Primers were designed with EcoRI recognition sequence on 5' end of the forward primer and XhoI recognition sequence on 5' end of the reverse primer to produce a PCR product, containing EcoRI and XhoI restriction sites on 5' and 3' ends respectively. Restriction sites were introduced in the positions allowing the fusion of the opened reading frame (ORF) of GL008 cDNA with 6xHis tag. Amplified products were controlled by performing agarose gel electrophoresis. PCR product of full length GL008 and pET28a+ vector were digested with EcoRI and XhoI. The total amount of digests were loaded on to the gel and separated by electrophoresis. The band with a size of approximately 1500 bp corresponding to the molecular weight of full length GL008 was cut out of the gel and DNA was extracted with GFX kit. Obtained fragment was cloned into pET28a+ vector and transformed into competent *E.coli* DH5a. Obtained clones were used for mini preparations of plasmid DNA.

### Miniprep DNA analysis

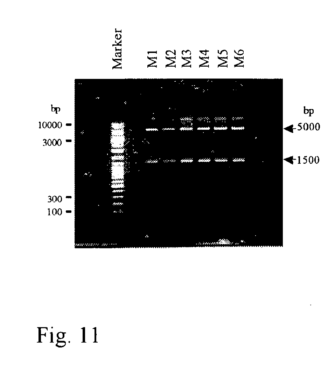

Plasmid DNA was isolated from the clones obtained after transformation. Clones were checked for the correct insert of GL008 by digesting the isolated plasmid DNA with EcoRI and XhoI. Digests were analyzed on 1.2 % agarose gel. Miniprep plasmid DNA M 1 (Fig. 11) containing pET28a-GL008 was retransformed in BL21 (DE3) RIL *E.coli* strain for the expression and purification of full length GL008 protein.

Fig. 11: Verification of pET28a-GL008 clones by electrophoresis.
M 1-M6 - mini prep DNA - pET28a-GL008.
Protein expression test for 6xHis-GL008 full length
Bacterial clones obtained after transformation with pET28a-GL008 were grown overnight in 5ml LB/kanamycin medium. On the next day, an aliquot of overnight culture was inoculated transferted in 7 ml of LB/kanamycin medium to obtain starting OD₆₀₀ of 0.1 - 0.15. Then the culture was grown until OD reached to 0.5 - 0.6. Bacteria were stimulated with IPTG for 3 hours. Stimulated bacteria were harvested and analyzed for the protein expression in comparison with non-stimulated ones by performing 15% SDS-PAGE and subsequent staining of the gel.

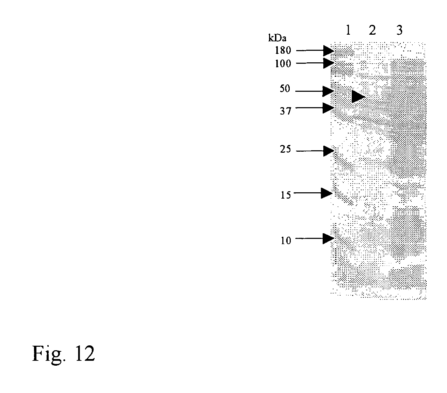

Fig. 12: SDS-PAGE analysis of 6xHis-GL008 expression in *E.coli*.
Lane 1: Marker
Lane 2: 6xHis-GL008 full length protein IPTG stimulated
Lane 3: 6xHis-GL008 full length protein non-stimulated

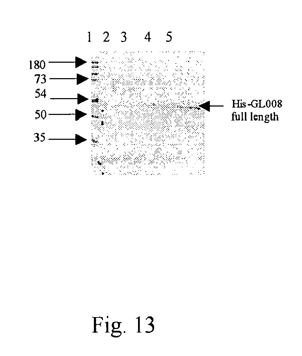

The protein band of approximately 50 kDa in lane 2 (marked) corresponds to 6xHis-GL008 full length protein (Fig. 12).
6xHis-GL008 purification conditions
Full length 6xHis-tagged GL008 was purified using Ni-NTA affinity resin as described in materials and methods. Initially, purification was carried out under denaturing conditions using 8M Urea. However SDS-PAGE analysis revealed that all the protein remained in pellet. Therefore denaturing with a buffer containing 6 M GuHCl was chosen. Protein purification was carried out and obtained protein was analyzed in Western blotting with anti-6xHis antibody. Protein band approximately at ~ 52 kDa corresponding to His-GL008 full length protein was detected (Fig. 13 lane 4 and 5), indicating efficiency and specificity of purification protocol.

Fig. 13: Western blot analysis of 6xHis-GL008 full length purification
Lane 1: Marker
Lane 2: elution fraction F2 from 8 M urea purification
Lane 3: elution fraction F3 from 8 M urea purification
Lane 4: elution fraction F2 from 6 M GuHCl purification
Lane 5: elution fraction F3 from GuHCl purification.
Expression of full length GL008-FLAG in mammalian cells
Cloning of plasmid pLP-IRESneo-GL008-FLAG was performed as part of my course work. Briefly, pLP-IRESneo is a mammalian expression vector in which GL008-FLAG tagged construct was cloned from pDNR2-GL008-FLAG by loxP - Cre-recombination.

### Transfection of CHO cells

CHO cells were transfected with pLP-IRESneo-GL008-FLAG plasmid. Transfection was carried out with 1µg and 3µg of pLP-IRESneo-GL008-FLAG plasmid and 1 µg of pLP-IRESneo as a control in six well plates as described in materials and methods.

### Western blotting analysis with anti-FLAG antibody

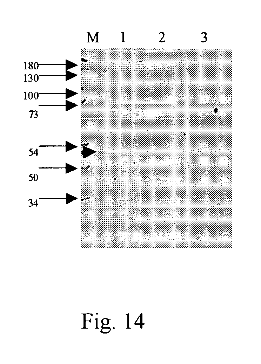

Transfected CHO cells were harvested 48 hours after transfection and 200-300 µl Laemmli sample buffer was added to lyse the cells. 25 µl of the cell lysate sample was loaded onto each lane, SDS-PAGE was carried out and gel was blotted onto nitrocellulose membrane. The expression of GL008-FLAG protein was detected with anti-FLAG antibody. CHO cells transfected with 1 µg of the plasmid expressed FLAG tagged GL008 protein (Fig 14, lane - 1 indicated with arrow).

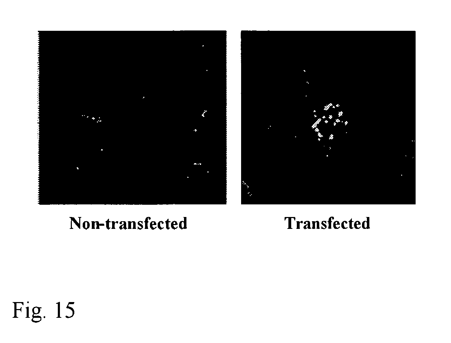

Fig. 14: Western blot analysis with anti-FLAG Ab
Lane 1 - transfected CHO cells with 1 µg plasmid
Lane 2 - transfected CHO cells with 3 µg plasmid
Lane 3 - non-transfected CHO cells.
Primary antibody: anti-FLAG Ab (1:1000 dilution).
Secondary antibody: Anti-mouse HRP conjugated (1:5000 dilution).
Immunofluorescence with anti-FLAG antibody
Six well plates with transfected CHO cells were fixed on glass cover slip as described. Prior to staining the samples were blocked with 3% BSA in PBS for 1 hour. For staining the samples were incubated with anti-FLAG antibody (1:500). Samples were then washed and incubated with anti-mouse Cy2 labeled (1:50) secondary antibody. Samples were mounted with immunofluorescence mounting medium (DakoCytomation) and analyzed by confocal microscopy. Anti-FLAG antibody recognized FLAG tagged GL008 protein expressed in mammalian cells (Fig. 15, transfected). Protein was localized in cytoplasmic granular structures.

Fig. 15: Immunofluorescence with anti-FLAG antibody
Cells were stained with anti-FLAG antibody followed by incubation with Cy2-conjugated anti-mouse secondary antibody. Green color corresponds to FLAG staining.

### Monoclonal anti-GL008 antibody analysis

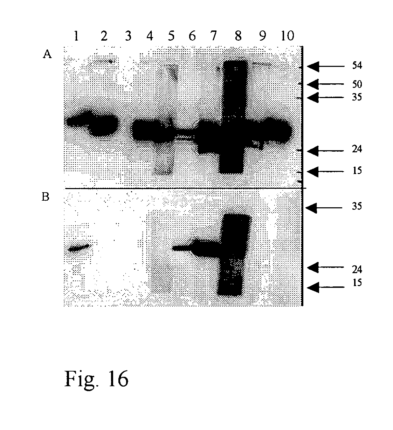

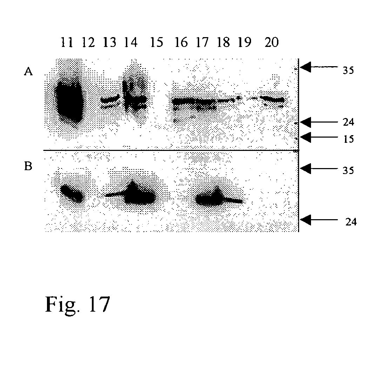

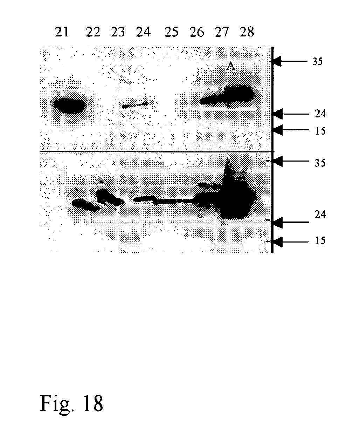

Western blot analysis with GST-GL008 and GST protein
GST-GL008-A and GST alone were loaded on different SDS-PAGE at a concentration of 100 ng per lane. The gels were blotted onto nitrocellulose membrane. Blot-A contained Gst-GL008-A protein and blot-B contained GST protein (Fig. 16). Blots were cut into stripes of one lane width. Stripe pairs (one containing GST-GL008-A and another containing GST alone) were incubated with hybridoma supernatants 1-10, followed with standard Western blotting detection (Fig. 16). The same procedure was followed to analyze hybridoma supernatants 11 to 20 (Fig. 17) and 21 to 28 (Fig. 18).

Fig. 16: Western blot analysis for hybridoma supernatants 1 - 10
A: antigen - GST-GL008-A; B: antigen - GST alone.
Lanes 1 to 10: supernatants 1 to 10 respectively, supernatants (primary Ab) dilution used - 1:3.
Secondary Ab: Anti-rat HRP labeled antibody (1:5000 dilution).

Fig. 17: Western blot analysis for hybridoma supernatants 11 - 20

A: antigen - GST-GL008-A; B: antigen ― GST alone.

Lanes 11 to 20: supernatants 11 to 20 respectively, supernatants (primary Ab) dilution used - 1:3

Secondary Ab: Anti-rat HRP labeled antibody (1:5000 dilution).

Fig. 18: Western blot analysis for hybridoma supernatants 21 - 28
A: antigen - GST alone; B: antigen - Gst-GL008-A.
Lanes 21 to 28: supernatants 21 to 28 respectively, supernatants (primary Ab) dilution used - 1:3
Secondary Ab: Anti-rat HRP labeled antibody (1:5000 dilution).

Hybridoma supernatants which reacted only with GST-GL008-A protein but with GST alone were selected as specific. Those supernatants were - 1, 2, 4, 5, 9, 10, 16, 19, 20, 22, 25, and 26.

### Western blot analysis with 6xHis-GL008 full length protein

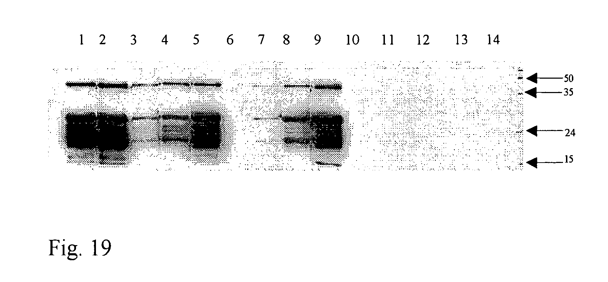

To analyze whether monoclonal antibody recognize the immunogen epitope in context to full length protein hybridoma supernatants specific to GST-GL008-A were tested against purified 6xHis-GL008 full length protein. Full length 6xHis-GL008 was loaded onto the gel at a concentration of 100 ng per lane, SDS-PAGE was carried out and gel was blotted onto the nitrocellulose membrane. Blots were cut into stripes of one lane width. Individual lanes were used for Western blot detection with supernatants specific to GL008-A (Fig. 19).

Fig. 19: Western blot analysis of hybridoma supernatants with His-GL008:
Antigen - 6xHis-GL008 full length.
Lanes 1 to 12: supernatants 1, 2, 4, 5, 9, 10, 16, 19, 20, 22, 25 and 26.
Lane 13: supernatant 15 (control - no signal to GL008 and GST)
Lane 14: supernatant 27 (control - GST positive)
Supernatants (primary antibody) dilution used - 1:3
Secondary antibody: Anti-rat HRP labeled antibody (1:5000 dilution).

Eight out of 12 supernatants recognized 6xHis-GL008 protein expressed in bacteria. These supernatants were: 2, 4, 5, 9, 10, 19, 20, and 22 (see Table 7). All 8 supernatants were further analyzed using GL008-FLAG tagged protein expressed in mammalian cells as an antigen.

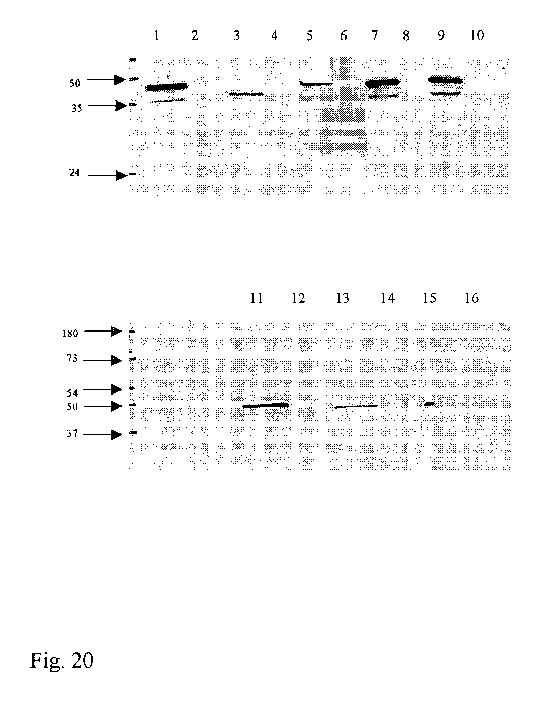

Western blot analysis with transfected cells
Supernatants 2, 4, 5, 9, 10, 19, 20, 22 were tested by Western blotting using total cell lysates of CHO-K1 and H1299 cells transfected with pLP-IRESneo-GL008-FLAG or an empty pLP-IRESneo vector as a negative control. Cells were transfected by lipofectamine technique, harvested 48 hours after transfection, and samples for SDS-PAGE were prepared by direct lysis of cell pellet in Laemmli buffer (200-300µl for 5.10⁵ cells). Protein lysates were separated in 15% SDS-PAGE and blotted onto nitrocellulose membrane. Individual lanes were cut from the membrane containing lysates of cells transfected with GL008 expression construct and empty vector. Two strips, one with GL008 and second vector control were incubated simultaneously with the same supernatant. Results of analysis of 8 supernatants are presented in Figure. 20.
Fig 20: Western blot analysis of hybridoma supernatants with transfected cells;
Antigen - Lanes 1, 3, 5, 7, 9, 11, 13, 15 - Transfected H1299 cells.
Lanes 2, 4, 6, 8, 10, 12, 14, 16- Empty vector (control).
Lane 1 and 2: supernatant 10
Lane 3 and 4: supernatant 9
Lane 5 and 6: supernatant 5
Lane 7 and 8: supernatant 4
Lane 9 and 10: supernatant 2
Lane 11 and 12: supernatant 22
Lane 13 and 14: supernatant 20
Lane 15 andl6: supernatant 19
Supernatants (primary Ab) dilution used - 1:3
Secondary antibody: Anti-rat HRP labeled antibody (1:5000 dilution)
All 8 supernatants recognized full length GL008 protein expressed in mammalian cells (Fig. 29). However supernatants 2, 4, 5 and 10 recognized specifically 2 bands and supernatants 9, 19, 20 and 22 recognized only one band. These two bands can be different forms of GL008 protein present in mammalian cells.
We concluded that all monoclonal antibodies, which reacted with bacterially purified full length GL008 protein, recognized the same protein expressed in mammalian cells, but with different epitopes.

### Immunofluorescence and confocal microscopy in transfected cells

The same 8 selected supernatants were tested for the ability to recognize GL008 full length protein in immunofluorescence. CHO-K1 and H1299 cells were transfected with pLP-IRESneo-GL008-FLAG or an empty pLP-IRESneo vector as a negative control. Thirty six hours after transfection the cells were fixed with PFA to ensure that intracellular vesicular structures remain intact. Permeabilisation step was included to provide access for antibodies to all intracellular structures. Blocking of the samples was performed in 3% BSA in PBS for 1 hour. Staining with primary antibodies was performed for 1 h 15 min with the combination of anti-FLAG (1:500) antibody and non-diluted hybridoma supernatants. After primary antibody incubation, cells were washed and incubated with secondary antibody. Anti-mouse-Cy2 and anti-rat-Cy3 antibodies were used at concentrations 1:50 and 1:200 respectively. For visualization of nucleus DAPI (1:1000) was added to secondary antibodies solution. After extensive washing in PBS, samples were mounted with immunofluorescence mounting medium and analyzed by confocal microscopy.

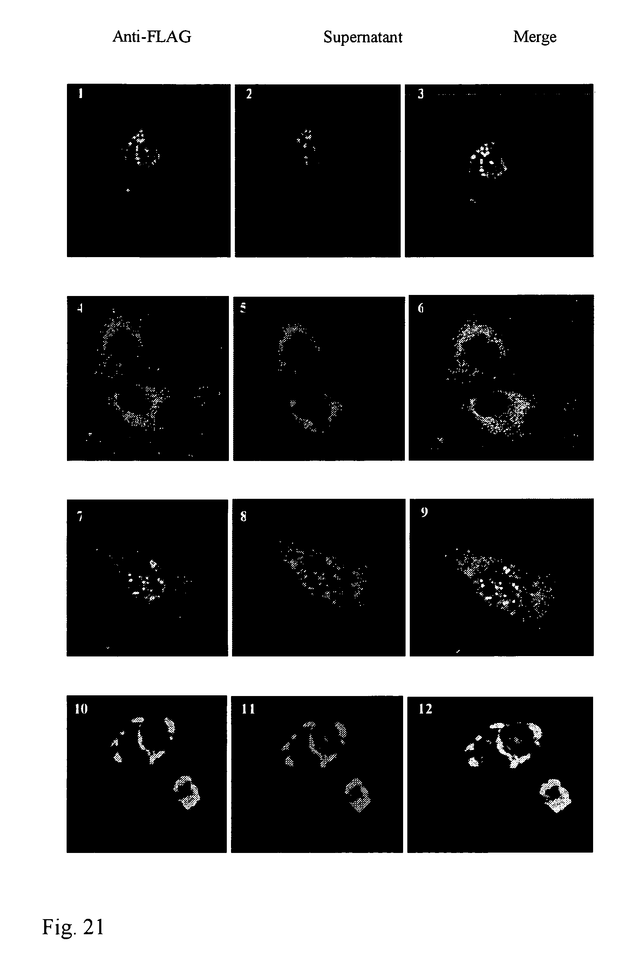

Fig. 21: Immunofluorescence in transfected CHO cells
Cells were stained with a combination of anti-FLAG Ab and hybridoma supernatant 2 (picture 1, 2, 3, 4, 5, and 6), hybridoma supernatant 5 (Picture 7, 8, and 9), hybridoma supernatant 9 (Picture 10, 11 and 12), followed by incubation with mixture of Cy2-conjugated anti-mouse and Cy3-conjugated anti-rat secondary antibody. Green color corresponds to FLAG staining, red color corresponds to GL008 staining and yellow color corresponds to overlap of FLAG and GL008.

The inventors observed that, all 8 supernatants recognized full length protein in immunofluorescence. Examples of staining are presented on Figure 21. Supernatant 2 recognized vesicular and dispersed structures (Fig. 21, pictures 1 to 6), supernatant 5 recognized vesicular structures (Fig. 21, pictures 7 to 9), and supernatant 9 recognized globular structures (Fig. 21, pictures 10-12).

Results of Western blotting and immunofluorescence for all supernatants tested are summarized in Table 3. Out of 8 clones positive in all tests, four were selected for the large scale production of monoclonal anti-GL008 antibody. These were clones 2; 4; 5 and 9 which recognized differential pattern of GL008 protein in Western blotting and immunofluorescence.

**Table 3. Summary of supernatants producing monoclonal anti-GL008 antibody**

| Supernatant original number | New number | GST-GL008 Positives | GST-Positives | 6xHis-GL008 positives | IF with Transfected cells(pLP-IRES-GL008-FLAG) | WB with Transfected cells (pLP-IRES-GL008-FLAG) |
|---|---|---|---|---|---|---|
| 1A7 | 1 | ++ | ++ | - | | |
| 1B8 | 2 | + + + | - | + + + | +++ | +++ |
| 1B9 | 3 | - | - | NT | | |
| 1C11 | 4 | +++ | - | +++ | +++ | +++ |
| 1D4 | 5 | + + | - | + + | +++ | + + |
| 1D11 | 6 | + + | ++ | NT | | |
| 1G8 | 7 | +++ | +++ | NT | | |
| 1H7 | 8 | +++ | +++ | NT | | |
| 2C2 | 9 | +++ | - | +++ | +++ | + + |
| 2C11 | 10 | +++ | - | +++ | +++ | +++ |
| 3D3 | 11 | ++ | ++ | NT | | |
| 3D6 | 12 | - | - | NT | | |
| 3D9 | 13 | ++ | ++ | NT | | |
| 3D11 | 14 | ++ | ++ | NT | | |
| 3E4 | 15 | - | - | - | | |
| 3F2 | 16 | ++ | - | - | | |
| 3F7 | 17 | ++ | ++ | NT | | |
| 3G7 | 18 | ++ | ++ | NT | | |
| 3H6 | 19 | +++ | - | + + | +++ | ++ |
| 4B3 | 20 | +++ | - | +++ | +++ | +++ |
| 4F5 | 21 | ++ | ++ | NT | | |
| 4G3 | 22 | +++ | - | +++ | +++ | +++ |
| 4H8 | 23 | - | - | - | | |
| 6C4 | 24 | ++ | ++ | NT | | |
| 6D11 | 25 | +++ | - | - | | |
| 7G12 | 26 | +++ | - | - | | |
| 7F11 | 27 | +++ | +++ | NT | | |
| 8H12 | 28 | +++ | +++ | NT | | |

| | | | | | | |
|---|---|---|---|---|---|---|
| "+++" very strongly positive | | | | | | |
| "++" strongly positive | | | | | | |
| "-" Negative or No reaction | | | | | | |
| NT Not tested | | | | | | |

Novel human chitinase like protein GL008 was identified as an interacting partner of Stabilin-1, a type I transmembrane receptor expressed on a subset of endothelial cells and type 2 macrophages. The fact, that expression of GL008 mRNA is up-regulated in Stabilin-1 positive macrophages prompted us to investigate the physiological significance of Stabilin-1/GL008 complex. Since Stabilin-1 was recently demonstrated to possess properties of endocytic and sorting receptor in macrophages (Kzhyshkowska, 2004), we hypothesized that interaction between GL008 and Stabilin-1 might occur in 2 physiological situations: 1) GL008 may serve as a cargo protein sorted by Stabilin-1 from biosynthetic compartments; 2) GL008 might be an extracellular ligand for Stabilin-1 mediated endocytosis. To examine these two possibilities, the first step was to generate anti-GL008 monoclonal antibodies. In the present work we described purification of a GL008 fragment for immunization, design of a multi-step test system for characterization of obtained monoclonal antibodies, and the analysis of their specificity.

### Test system for developed antibody

To confirm that hybridoma supernatants specifically react with GST-GL008-A fragment they were tested in Western blot using recombinant GST-GL008-A and GST alone. It was found that only 12 out of 28 clones specifically recognise GST-GL008-A in Western blot. These 12 clones were further tested to recognise full-length recombinant GL008 in Western blot. We also used 6xHis-tagged GL008 to confirm that the antibody recognise GL008 itself and not its combination with GST. Eight out of 12 clones appeared to recognise bacterially expressed GL008 protein specifically. Since *E.coli* expressed protein may lack several mammalian-specific protein modifications we next tested whether selected eight hybridoma supernatants recognise GL008 protein expressed in mammalian cells. Hamster ovarian fibroblasts CHO-K1 and human lung carcinoma H1299 cells were selected for transient protein expression due to their high transfection efficiency. These cells were transiently transfected with expression construct for full length GL008 fused to FLAG epitope on its C-terminus. Efficiency of GL008 expression in these cells was confirmed with anti-FLAG antibody in Western blotting and indirect immunofluorescence. Western blotting with 8 hybridoma supernatants revealed that they all recognise GL008-FLAG expressed in mammalian cells. Surprisingly, supernatants 2, 4, 5 and 10 recognized two forms of GL008 expressed in transiently transfected H1299 cells (Fig 30, lanes 9, 7, 5 and 1 respectively). The upper protein band (~ 50 kDa) in all the lanes might correspond to full length protein, which MW was predicted to be 45 kDa; the lower band of about 35 kDa might be a result of proteolytic processing of GL008 protein. Proteolytic post-translational cleavage was described for other Glyco_18 domain containing mammalian protein chitotriosidase that is secreted as 50kDa active enzyme, but its truncated 39kDa version is accumulated in macrophage lysosomes. Proteolytic cleavage occurs on the C-terminal of the protein (51;61) (43). Remaining 4 hybridoma supernatants recognized only the upper band. Next we analyzed the hybridoma supernatants in indirect immunofluorescence assay. Transiently transfected cells were fixed with PFA providing that all intracellular membrane structures remain intact. Similarly to Western blot results, supernatants recognized different patterns of protein localization in immunofluorescence assay in transfected cells. Supernatant 2 recognized vesicular structures and dispersed structures; supernatant 9 recognized globular protein structures, suggesting that GL008 protein may form crystals in the cell. To confirm the specificity of supernatants tested a double immunofluorescence with anti-GL008 hybridoma supernatants and anti-FLAG antibody was performed. Hybridoma supernatants and anti-FLAG antibody stained the same structures in transiently transfected cells, however supernatant 5 revealed some structures not stained with a-FLAG antibody, suggesting that the C-terminal of GL008 may be processed post-translationally similarly to that of human chitotriosidase.

Thus, GL008-A protein fragment with 50 amino acids was immunogenic resulted in generation of 8 highly specific monoclones against GL008. These monoclones recognized not only bacterially expressed GL008-A, but also a full-length protein expressed in mammalian cells. All specific monoclones recognized both denatured antigen in Western blotting and the antigen in native conformation in PFA fixed mammalian cells.

Further analysis is required to analyze whether new monoclonal antibodies recognize GL008 antigen in cells and tissues under different fixation conditions including acetone, methanol, and ethanol. To study association of GL008 with human disorders antibody should be tested to work in paraffin embedded material. To perform functional studies several other properties of novel antibodies have to be characterized including their activity in immunoprecipitation and their blocking properties in protein-protein binding studies.

### Application of generated antibodies for functional analysis of GL008

Highly specific monoclonal antibodies recognizing novel human chitinase-like GL008 protein can be used to study function of the endogenous GL008 in human macrophages. First, the presence and differential expression of GL008 protein in macrophages subpopulations has to be confirmed by immunofluorescence and quantitative Western blot analysis. The ability of different monoclones to recognise different forms of GL008 protein in mammalian cells enable the analysis of posttranslational processing and intracellular localization of different GL008. Co-localization of Stabilin-1 and GL008 in human macrophages can be analysed using double immunofluorescence and confocal microscopy. Triple staining for stabilin-1, GL008 and markers for endosomal/lysosomal compartments and secretory pathway will be performed to analyze the physiological significance of satbilin-1/GL008 complex. In summary, generated novel monoclonal antibodies is a tool to study function of novel human putative chitinase-like protein.

### Analysis of GL008 expression in human primary macrophages

### Cells and mediators

The isolation and cultivation of human monocytes/macrophages was done according following protocol. The cells were purified from individual buffy coats. Buffy coats were diluted with Ca²⁺- and Mg²⁺- free phosphate-buffered saline (PBS) (Biochrom) at a ratio 1:1. A total of 35 ml of diluted buffy coats were layered on top of 15 ml Ficoll-Paque (Biochrom, Berlin, Germany) in a 50-ml Leucosep tube (Greiner, Fickenhausen, Germany). After 30 min of centrifugation in a swing out rotor (Beckman Coulter, Fullerton, CA, USA) at 650xg, peripheral blood mononuclear cells (PBMC) were collected from the Ficoll serum interphase. PBMCs were washed two times with PBS (Biochrom). The Percoll gradient was preformed by centrifugation of freshly prepared Percoll (13.5 ml Percoll (Pharmacia, Freiburg, Germany), 1.5 ml 10 x Earle's Minimal Essential Medium (MEM), 15 ml Spinner's medium) at 12000xg for 10 min at 20 °C without breaks in a F34-6-38 rotor (Eppendorf, Hamburg, Germany). Five to 8x10⁸ PBMCs were layered on top of the Percoll gradient and centrifuged at 650xg for 30 min at 20 °C without breaks. The upper layer, containing 60-80% monocytes was collected and washed three times with PBS. The cell population obtained after Percoll gradient centrifugation was subjected to CD14+ magnetic cell sorting using monocyte isolation kit (Miltenyi Biotech, Bergisch Gladbach, Germany), resulting in 92-98% monocyte purity, controlled by flow cytometry. Macrophages were cultured at 5x10⁵ cell/mL in X-vivo 10 serum free medium (Cambrex, Verviers, Belgium), supplemented with cytokines and/or dexamethasone as indicated, for 6 days to obtain fully established phenotype.

Human interferon (IFN) y, from TEBU Peprotech (Frankfurt am Main, Germany), was used at a concentration of 1000 U/ml. IL-4 and TGF β were from TEBU Peprotech, and were used at a concentration of 10 ng/ml. Dexamethasone was used at 1x10⁻⁷ M.

### RNA isolation and cDNA synthesis

For RNA isolation, the cells were lysed directly in the plastic Petri dishes and the RNA was isolated using RNeasy Mini kit (Qiagen, Hilden, Germany) according to the recommendations of the manufacturer.

Total RNA (2 µg) was treated with 2U RNase free DNase (Ambion, Austin, TX, USA) and used for Reverse Transcription (RT) with Superscript II reverse transcriptase (Invitrogen, Karlsruhe, Gemany) using oligo dT primers according to the recommendations of the manufacturer. The obtained cDNA was diluted 1:10 with DDH2O and 1 µl was used for PCR reaction.

### Real-Time RT-PCR analysis

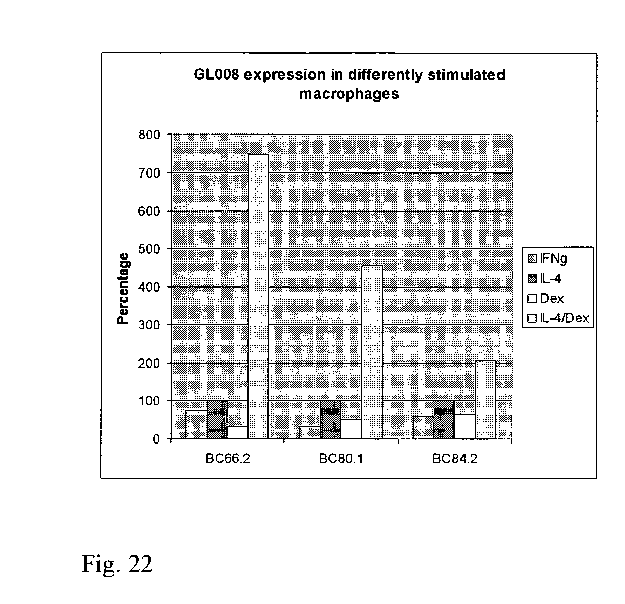

Real time PCR analysis of GL008 expression was performed using SYBR Green PCR master mix (Applied Biosystems, Darmstadt, Germany) using standard conditions. Primer sequences are F591 ACGCACAAGGAGTTTGAGCAG, R591 GGATTTTGCTTCGCCACTTG. As internal control a TaqMan probe for human GAPDH was used together with TaqMan PCR master mix (all from Applied Biosystems). The experiments were performed on ABI PRISM® 7000 sequence detection system (Applied Biosystems). The expression levels of analysed genes were normalized to GAPDH mRNA expression. The level of GL008 mRNA up-regulation was donor-dependent (see Figure 22).

### Western blot analysis

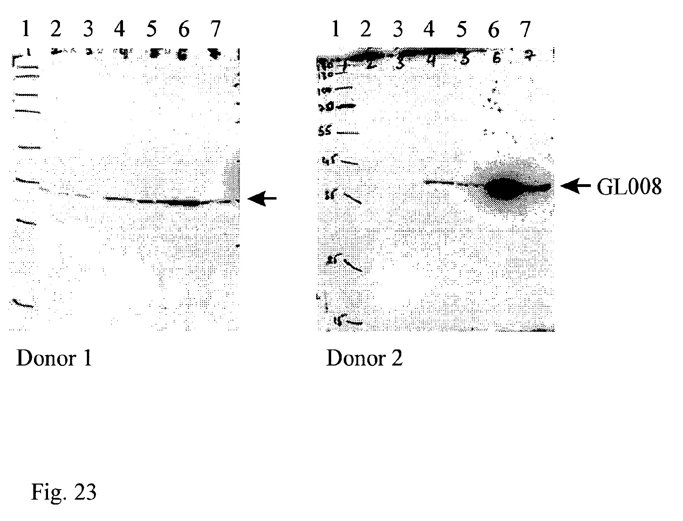

Macrophage samples were re-suspended in Laemmli sample buffer (200µL of sample buffer for 5 x10⁵ cells). After heating at 95°C for 5 min, 25µL of each sample were analysed by Western blotting. Amount of the protein loaded on the gel was controlled by Gel Code reagent (Pierce) staining. Primary ab was rat monoclonal anti-GL008 clone 1C11, secondary anti-rat HRP linked (Amersham). Following stimulations were used: 1) non-stimulated human macrophages; 2) IFNgamma; 3) IL4; 4) dexamethasone only; 5) IL4 + dexamethasone; 6) IL4+TGFbeta. Up-regulation of GL008 protein expression by Th2 cytokines and dexamethasone was observed in all tested donors, but the level of up-regulation was donor- specific. Important, that dexamethasone alone has significantly less effect on GL008 expression. This fact is related to high apoptotic ratio in human dexamethasone-treated macrophage culture without addition of cytokines. Dramatic over-expression of GL008 in macrophages stimulated by IL4 and dexamethasone indicates, that IL4-dependent viability of macrophages is needed to respond to dexamethasone. Combination of IL4+dexamethasone corresponds to the natural situation of Th2-associated inflammation/allergy, which is typically treated by corticoids, endogenous and exogenous corticoids act always in combination with other cytokines in human tissues. Two examples of weak reaction (donor 1) and strong reaction (donor 2) are presented on Figure 23.

In summary, both analysis of mRNA and protein level indicate that dexamethasone in combination with Th2 cytokine up-regulates GL008 expression in donor-dependent manner.

### Figure 22. Real-time PCR analysis of GL008 mRNA expression in human primary macrophages under different stimulations

### Figure 23. Western blotting analysis of GL008 protein expression in human primary macrophages under different stimulations

Lane 1: protein markers
Lane 2: non-stimulated human macrophages
Lane 3: IFNgamma
Lane 4: IL4
Lnae 5: dexamethasone only
Lane 6: IL4 + dexamethasone
Lane 7: IL4+TGFbeta

In the present application, the inventors describe the purification of immunogenic fragment of novel human chitinase-like GL008 proteins, which was used for rat immunization. Using a multi-step test system we selected and characterized 8 monoclonal antibodies, which can be used for Western blotting and immunofluorescence identification of GL008 protein expressed in mammalian cells. Immunofluorescence analysis revealed that GL008 is localized in large intracellular structures in transiently transected mammalian cells. Novel monoclonal antibodies are a highly specific tool to study function of chitinase-like protein in *in vitro* and *in vivo* systems and characterize physiological significance of GL008/Stabilin-1 complex.

References
(1) Burke B, Lewis C. The Macrophage. Oxford: Oxford University press, 2002.
(2) van Rooijen N, Wijburg OL, van den Dobbelsteen GP, Sanders A. Macrophages in host defense mechanisms. Curr Top Microbiol Immunol 1996; 210:159-165.
(3) Bosque F, Belkaid Y, Briend E, Hevin B, Lebastard M, Soussi N et al. The biology of macrophages. Pathol Biol (Paris) 1997; 45(2):103-109.
(4) Goerdt S, Politz O, Schledzewski K, Birk R, Gratchev A, Guillot P et al. Alternative versus classical activation of macrophages. Pathobiology 1999; 67(5-6):222-226.
(5) Stout RD, Suttles J. T cell signaling of macrophage function in inflammatory disease. Front Biosci 1997; 2:d197-d206.
(6) Aderem A, Underhill DM. Mechanisms of phagocytosis in macrophages. Annu Rev Immunol 1999; 17:593-623.
(7) Underhill DM, Ozinsky A. Phagocytosis of microbes: complexity in action. Annu Rev Immunol 2002; 20:825-852.
(8) Adams DO, Hamilton TA. The cell biology of macrophage activation. Annu Rev Immunol 1984; 2:283-318.
(9) Goerdt S, Orfanos CE. Other functions, other genes: alternative activation of antigen-presenting cells. Immunity 1999; 10(2):137-142.
(10) Gratchev A, Schledzewski K, Guillot P, Goerdt S. Alternatively activated antigen-presenting cells: molecular repertoire, immune regulation, and healing. Skin Pharmacol Appl Skin Physiol 2001; 14(5):272-279.
(11) Gordon S. Alternative activation of macrophages. Nat Rev Immunol 2003; 3(1):23-35.
(12) Mantovani A, Sozzani S, Locati M, Allavena P, Sica A. Macrophage polarization: tumor-associated macrophages as a paradigm for polarized M2 mononuclear phagocytes. Trends Immunol 2002; 23(11):549-555.
(13) Stein M, Keshav S, Harris N, Gordon S. Interleukin 4 potently enhances murine macrophage mannose receptor activity: a marker of alternative immunologic macrophage activation. J Exp Med 1992; 176(1):287-292.
(14) Mosser DM, Handman E. Treatment of murine macrophages with interferon-gamma inhibits their ability to bind leishmania promastigotes. J Leukoc Biol 1992; 52(4):369-376.
(15) Geng YJ, Hansson GK. Interferon-gamma inhibits scavenger receptor expression and foam cell formation in human monocyte-derived macrophages. J Clin Invest 1992; 89(4):1322-1330.
(16) Hogger P, Dreier J, Droste A, Buck F, Sorg C. Identification of the integral membrane protein RM3/1 on human monocytes as a glucocorticoid-inducible member of the scavenger receptor cysteine-rich family (CD163). J Immunol 1998; 161(4):1883-1890.
(17) Kristiansen M, Graversen JH, Jacobsen C, Sonne O, Hoffman HJ, Law SK et al. Identification of the haemoglobin scavenger receptor. Nature 2001; 409(6817): 198-201.
(18) Goerdt S, Kolde G, Bonsmann G, Hamann K, Czarnetzki B, Andreesen R et al. Immunohistochemical comparison of cutaneous histiocytoses and related skin disorders: diagnostic and histogenetic relevance of MS-1 high molecular weight protein expression. J Pathol 1993; 170(4):421-427.
(19) Szekanecz Z, Haines GK, Lin TR, Harlow LA, Goerdt S, Rayan G et al. Differential distribution of intercellular adhesion molecules (ICAM-1, ICAM-2, and ICAM-3) and the MS-1 antigen in normal and diseased human synovia. Their possible pathogenetic and clinical significance in rheumatoid arthritis. Arthritis Rheum 1994; 37(2):221-231.
(20) Djemadji-Oudjiel N, Goerdt S, Kodelja V, Schmuth M, Orfanos CE. Immunohistochemical identification of type II alternatively activated dendritic macrophages (RM 3/1+3, MS-1+/-, 25F9-) in psoriatic dermis. Arch Dermatol Res 1996; 288(12):757-764.
(21) Schebesch C, Kodelja V, Muller C, Hakij N, Bisson S, Orfanos CE et al. Alternatively activated macrophages actively inhibit proliferation of peripheral blood lymphocytes and CD4+ T cells *in vitro*. Immunology 1997; 92(4):478-486.
(22) Cheung DL, Hart PH, Vitti GF, Whitty GA, Hamilton JA. Contrasting effects of interferon-gamma and interleukin-4 on the interleukin-6 activity of stimulated human monocytes. Immunology 1990; 71(1):70-75.
(23) Standiford TJ, Kunkel SL, Liebler JM, Burdick MD, Gilbert AR, Strieter RM. Gene expression of macrophage inflammatory protein-1 alpha from human blood monocytes and alveolar macrophages is inhibited by interleukin-4. Am J Respir Cell Mol Biol 1993; 9(2):192-198.
(24) Goerdt S, Walsh LJ, Murphy GF, Pober JS. Identification of a novel high molecular weight protein preferentially expressed by sinusoidal endothelial cells in normal human tissues. J Cell Biol 1991; 113(6):1425-1437.
(25) Goerdt S, Bhardwaj R, Sorg C. Inducible expression of MS-1 high-molecular- weight protein by endothelial cells of continuous origin and by dendritic cells/macrophages *in vivo* and *in vitro*. Am J Pathol 1993; 142(5):1409-1422.
(26) Walsh LJ, Goerdt S, Pober JS, Sueki H, Murphy GF. MS-1 sinusoidal endothelial antigen is expressed by factor XIIIa+, HLA-DR+ dermal perivascular dendritic cells. Lab Invest 1991; 65(6):732-741.
(27) Politz O, Gratchev A, McCourt PA, Schledzewski K, Guillot P, Johansson S et al. Stabilin-1 and -2 constitute a novel family of fasciclin-like hyaluronan receptor homologues. Biochem J 2002; 362(Pt 1):155-164.
(28) Hawtin RE, Arnold K, Ayres MD, Zanotto PM, Howard SC, Gooday GW et al. Identification and preliminary characterization of a chitinase gene in the Autographa califomica nuclear polyhedrosis virus genome. Virology 1995; 212(2):673-685.
(29) Vinetz JM, Dave SK, Specht CA, Brameld KA, Xu B, Hayward R et al. The chitinase PfCHT1 from the human malaria parasite Plasmodium falciparum lacks proenzyme and chitin-binding domains and displays unique substrate preferences. Proc Natl Acad Sci U S A 1999; 96(24):14061-14066.
(30) Brurberg MB, Nes IF, Eijsink VG. Comparative studies of chitinases A and B from Serratia marcescens. Microbiology 1996; 142 ( Pt 7):1581-1589.
(31) Kuranda MJ, Robbins PW. Chitinase is required for cell separation during growth of Saccharomyces cerevisiae. J Biol Chem 1991; 266(29):19758-19767.
(32) Flach J, Pilet PE, Jolles P. What's new in chitinase research? Experientia 1992; 48(8):701-716.
(33) Sakuda S, Isogai A, Matsumoto S, Suzuki A. Search for microbial insect growth regulators. n. Allosamidin, a novel insect chitinase inhibitor. J Antibiot (Tokyo) 1987; 40(3):296-300.
(34) Henrissat B, Bairoch A. New families in the classification of glycosyl hydrolases based on amino acid sequence similarities. Biochem J 1993; 293 ( Pt 3):781-788.
(35) Terwisscha van Scheltinga AC, Hennig M, Dijkstra BW. The 1.8 A resolution structure of hevamine, a plant chitinase/lysozyme, and analysis of the conserved sequence and structure motifs of glycosyl hydrolase family 18. J Mol Biol 1996; 262(2):243-257.
(36) Coulson AF. A proposed structure for 'family 18' chitinases. A possible function for narbonin. FEBS Lett 1994; 354(1):41-44.
(37) Owhashi M, Arita H, Hayai N. Identification of a novel eosinophil chemotactic cytokine (ECF-L) as a chitinase family protein. J Biol Chem 2000; 275(2): 1279-1286.
(38) Renkema GH, Boot RG, Muijsers AO, Donker-Koopman WE, Aerts JM. Purification and characterization of human chitotriosidase, a novel member of the chitinase family of proteins. J Biol Chem 1995; 270(5):2198-2202.
(39) Renkema GH, Boot RG, Au FL, Donker-Koopman WE, Strijland A, Muijsers AO et al. Chitotriosidase, a chitinase, and the 39-kDa human cartilage glycoprotein, a chitin-binding lectin, are homologues of family 18 glycosyl hydrolases secreted by human macrophages. Eur J Biochem 1998; 251(1-2):504-509.
(40) Lok IH, Briton-Jones CM, Yuen PM, Haines CJ. Variable expression of oviductin mRNA at different stages of human reproductive cycle. J Assist Reprod Genet 2002; 19(12):569-576.
(41) Lapensee L, Paquette Y, Bleau G. Allelic polymorphism and chromosomal localization of the human oviductin gene (MUC9). Fertil Steril 1997; 68(4):702-708.
(42) Arias EB, Verhage HG, Jaffe RC. Complementary deoxyribonucleic acid cloning and molecular characterization of an estrogen-dependent human oviductal glycoprotein. Biol Reprod 1994; 51(4):685-694.
(43) Boot RG, Blommaart EF, Swart E, Ghauharali-van der Vlugt K, Bijl N, Moe C et al. Identification of a novel acidic mammalian chitinase distinct from chitotriosidase. J Biol Chem 2001; 276(9):6770-6778.
(44) Debono M, Gordee RS. Antibiotics that inhibit fungal cell wall development. Annu Rev Microbiol 1994; 48:471-497.
(45) Tjoelker LW, Gosting L, Frey S, Hunter CL, Trong HL, Steiner B et al. Structural and functional definition of the human chitinase chitin-binding domain. J Biol Chem 2000; 275(1):514-520.
(46) Hollak CE, van Weely S, van Oers MH, Aerts JM. Marked elevation of plasma chitotriosidase activity. A novel hallmark of Gaucher disease. J Clin Invest 1994; 93(3):1288-1292.
(47) Steck E, Breit S, Breusch SJ, Axt M, Richter W. Enhanced expression of the human chitinase 3-like 2 gene (YKL-39) but not chitinase 3-like 1 gene (YKL-40) in osteoarthritic cartilage. Biochem Biophys Res Commun 2002; 299(1):109-115.
(48) Steenbakkers PJ, Harhangi HR, Bosscher MW, van der Hooft MM, Keltjens JT, van der DC et al. beta-Glucosidase in cellulosome of the anaerobic fungus Piromyces sp. strain E2 is a family 3 glycoside hydrolase. Biochem J 2003; 370(Pt 3):963-970.
(49) Shostak K, Labunskyy V, Dmitrenko V, Malisheva T, Shamayev M, Rozumenko V et al. HC gp-39 gene is upregulated in glioblastomas. Cancer Lett 2003; 198(2):203-210.
(50) Kronborg G, Ostergaard C, Weis N, Nielsen H, Obel N, Pedersen SS et al. Serum level of YKL-40 is elevated in patients with Streptococcus pneumoniae bacteremia and is associated with the outcome of the disease. Scand J Infect Dis 2002; 34(5):323-326.
(51) Boot RG, Renkema GH, Strijland A, van Zonneveld AJ, Aerts JM. Cloning of a cDNA encoding chitotriosidase, a human chitinase produced by macrophages. J Biol Chem 1995; 270(44):26252-26256.
(52) Renkema GH, Boot RG, Strijland A, Donker-Koopman WE, van den BM, Muijsers AO et al. Synthesis, sorting, and processing into distinct isoforms of human macrophage chitotriosidase. Eur J Biochem 1997; 244(2):279-285.
(53) Aguilera B, Ghauharali-van der Vlugt K, Helmond MT, Out JM, Donker-Koopman WE, Groener JE et al. Transglycosidase activity of chitotriosidase: improved enzymatic assay for the human macrophage chitinase. J Biol Chem 2003; 278(42):40911-40916.
(54) Boot RG, Renkema GH, Verhoek M, Strijland A, Bliek J, de Meulemeester TM et al. The human chitotriosidase gene. Nature of inherited enzyme deficiency. J Biol Chem 1998; 273(40):25680-25685.
(55) Saito A, Ozaki K, Fujiwara T, Nakamura Y, Tanigami A. Isolation and mapping of a human lung-specific gene, TSA1902, encoding a novel chitinase family member. Gene 1999; 239(2):325-331.
(56) Chang NC, Hung SI, Hwa KY, Kato I, Chen JE, Liu CH et al. A macrophage protein, Ym1, transiently expressed during inflammation is a novel mammalian lectin. J Biol Chem 2001; 276(20):17497-17506.
(57) Harbord M, Novelli M, Canas B, Power D, Davis C, Godovac-Zimmermann J et al. Ym1 is a neutrophil granule protein that crystallizes in p47phox-deficient mice. J Biol Chem 2002; 277(7):5468-5475.
(58) Welch JS, Escoubet-Lozach L, Sykes DB, Liddiard K, Greaves DR, Glass CK. TH2 cytokines and allergic challenge induce Ym1 expression in macrophages by a STAT6-dependent mechanism. J Biol Chem 2002; 277(45):42821-42829.
(59) Hu B, Trinh K, Figueira WF, Price PA. Isolation and sequence of a novel human chondrocyte protein related to mammalian members of the chitinase protein family. J Biol Chem 1996; 271(32):19415-19420.
(60) Knorr T, Obermayr F, Bartnik E, Zien A, Aigner T. YKL-39 (chitinase 3-like protein 2), but not YKL-40 (chitinase 3-like protein 1), is up regulated in osteoarthritic chondrocytes. Ann Rheum Dis 2003; 62(10):995-998.
(61) Fusetti F, von Moeller H, Houston D, Rozeboom HJ, Dijkstra BW, Boot RG et al. Structure of human chitotriosidase. Implications for specific inhibitor design and function of mammalian chitinase-like lectins. J Biol Chem 2002; 277(28):25537-25544.
(62) Nishikawa KC, Millis AJ. gp38k (CHI3L1) is a novel adhesion and migration factor for vascular cells. Exp Cell Res 2003; 287(1):79-87.
(63) Recklies AD, White C, Ling H. The chitinase 3-like protein human cartilage glycoprotein 39 (HC-gp39) stimulates proliferation of human connective-tissue cells and activates both extracellular signal-regulated kinase- and protein kinase B-mediated signalling pathways. Biochem J 2002; 365(Pt 1): 119-126.
(64) Rehli M, Krause SW, Andreesen R. Molecular characterization of the gene for human cartilage gp-39 (CHI3L1), a member of the chitinase protein family and marker for late stages of macrophage differentiation. Genomics 1997; 43(2):221-225.
(65) Hakala BE, White C, Recklies AD. Human cartilage gp-39, a major secretory product of articular chondrocytes and synovial cells, is a mammalian member of a chitinase protein family. J Biol Chem 1993; 268(34):25803-25810.
(66) Morrison BW, Leder P. neu and ras initiate murine mammary tumors that share genetic markers generally absent in c-myc and int-2-initiated tumors. Oncogene 1994; 9(12):3417-3426.
(67) Nair MG, Cochrane DW, Allen JE. Macrophages in chronic type 2 inflammation have a novel phenotype characterized by the abundant expression of Ym1 and Fizz1 that can be partly replicated *in vitro.* Immunol Lett 2003; 85(2):173-180.
(68) Raes G, Noel W, Beschin A, Brys L, de Baetselier P, Hassanzadeh GH. FIZZ 1 and Ym as tools to discriminate between differentially activated macrophages. Dev Immunol 2002; 9(3):151-159.
(69) Raes G, de Baetselier P, Noel W, Beschin A, Brombacher F, Hassanzadeh GG. Differential expression of FIZZI and Ym1 in alternatively versus classically activated macrophages. J Leukoc Biol 2002; 71(4):597-602.
(70) Hung SI, Chang AC, Kato I, Chang NC. Transient expression of Ym1, a heparin-binding lectin, during developmental hematopoiesis and inflammation. J Leukoc Biol 2002; 72(1):72-82.
(71) Sendai Y, Komiya H, Suzuki K, Onuma T, Kikuchi M, Hoshi H et al. Molecular cloning and characterization of a mouse oviduct-specific glycoprotein. Biol Reprod 1995; 53(2):285-294.
(72) Takahashi K, Sendai Y, Matsuda Y, Hoshi H, Hiroi M, Araki Y. Mouse oviduct-specific glycoprotein gene: genomic organization and structure of the 5'-flanking regulatory region. Biol Reprod 2000; 62(2):217-226.
(73) Araki Y, Nohara M, Yoshida-Komiya H, Kuramochi T, Ito M, Hoshi H et al. Effect of a null mutation of the oviduct-specific glycoprotein gene on mouse fertilization. Biochem J 2003; 374(Pt 2):551-557.

## Claims

1. A binding agent capable of binding to a protein comprising the amino acid sequence of SEQ ID NO: 1 or to a variant of said sequence, wherein said variant comprises one or more insertions, substitutions and/or deletions as compared to the sequence of SEQ ID NO: 1, and wherein the biological activity of the variant is substantially equal to the activity of the protein comprising the unmodified amino acid sequence of SEQ ID NO: 1.

2. A binding agent of claim 1, which is capable of specifically binding to a sequence corresponding to amino acids 24-73 of SEQ ID NO: 1.

3. A binding agent capable of binding to a protein encoded by the nucleic acid sequence of SEQ ID NO: 2 or variants thereof, wherein the variants are each defined as having one or more substitutions, insertions and/or deletions as compared to the sequence of SEQ ID NO: 2, provided that said variants hybridize under moderately stringent conditions to a nucleic acid which comprises the sequence of SEQ ID NO: 2 and further provided that said variants code for a protein with an activity equal to the activity of the protein comprising the unmodified amino acid sequence encoded by SEQ ID NO: 2 or provided that said variants comprise nucleic acid changes due to the degeneracy of the genetic code, which code for the same or a functionally equivalent amino acid as the nucleic acid sequence of SEQ ID NO: 2.

4. The binding agent of one or more of claims 1-3, wherein the binding agent is an antibody or an aptamer.

5. The binding agent of claim 4, wherein said antibody is selected from the group consisting of a polyclonal antibody, a monoclonal antibody, a humanized antibody, a chimeric antibody, and a synthetic antibody.

6. The binding agent of claim 4 or 5 wherein said antibody is linked to a toxic agent and/or to a detectable agent.

7. A hybridoma which produces a monoclonal antibody of claim 5.

8. An inhibitor of the protein as defined in claims 1-3, wherein said inhibitor is selected from the group consisting of a molecule that reduces the level of mRNA encoding said protein, a molecule that reduces the level of said protein, and a molecule that reduces the biological activity of said protein.

9. The inhibitor of claim 8, wherein said inhibitor is selected from the group consisting of an antisense nucleic acid, a ribozyme, double stranded RNA, an antibody, a peptide and a peptidomimetic.

10. A nucleic acid, which selectively hybridizes to the nucleic acid of claim 3 or transcriptional products thereof under moderately stringent conditions.

11. The nucleic acid of claim 10, which is anti-sense DNA or RNA or a DNA- or RNA-probe.

12. A nucleic acid, which is a transcriptional product of the nucleic acid as defined in claim 3.

13. A vector, which comprises the nucleic acid of any of claims 3 or 10-12.

14. An expression vector, which comprises the nucleic acid of any of claim 3 or 10-12 and one or more regulatory sequences, wherein the vector preferably is a plasmid.

15. A host cell, which has been transformed with the vector of claim 13 or 14.

16. The host cell of claim 15, which is a eucaryotic cell, preferably a mammalian cell, plant cell, yeast cell, or an insect cell, wherein the mammalian cell more preferably is a *CHO-, COS-, HeLa-, 293T-, HEH-,* or *BHK-cell* or an adult or embryonic stem cell.

17. The host cell of claim 15, which is a procaryotic cell, preferably *E.coli* or *Bacillus subtilis.*

18. A pharmaceutical or diagnostic composition, comprising a binding agent of one or more of claims 1-6 or an inhibitor of claims 8 or 9 optionally in combination with a pharmaceutically acceptable carrier and/or diluent and/or auxiliary materials.

19. The use of a binding agent of one or more of claims 1-6, of an inhibitor of one or more of claims 7 and 8 or of a composition of claim 18 for the manufacture of a medicament for the treatment of inflammation/allergies/asthma, for alleviating effects associated with glucocorticoid therapy or for the treatment of cancer.

20. The use of a binding agent of one or more of claims 1-6 or of a composition of claim 18 for the diagnosis and/or determination of individual reactivity of patients on glucocorticoid therapy, diagnosis of inflammation/allergies/asthma, prognosis of glucocorticoid effects and for the diagnosis/prognosis of cancer.

21. An *ex vivo* method for the diagnosis and/or determination of individual reactivity of patients to glucocorticoid therapy, diagnosis of inflammation/allergies/asthma, prognosis of glucocorticoid effects and for the diagnosis/prognosis of cancer comprising the steps of:
a) providing a patient sample;
b) qualitative and/or quantitative determination of the amount of protein according to SEQ ID NO: 1, of the nucleic acid according to SEQ ID NO: 2 or translational products thereof in this sample;
c) wherein the amount of protein or nucleic acid as determined in b) being indicative of the reactivity of said patient on glucocorticoid therapy, of inflammation/allergies, of glucocorticoid effects or of cancer.

22. The method of claim 21, wherein the patient sample contains primary and cultured monocytes and macrophages.

23. The methods of claims 21 or 22, wherein the determination in step b) is performed by means of a binding agent of one or more of claim 1-6 and/or by means of Western Blotting, RT-PCR, immunohistologic determination, immunoassay or a combination thereof.

24. A method of making an isolated protein as defined in one of claims 1-3 comprising (a) culturing any one of the host cells of claim 15-17 under conditions such that the protein is expressed; and (b) recovering said protein.

25. A method of determining a candidate compound's ability of causing an allergy, inflammation, asthma or cancer, or for determining glucocorticoid effects in a non-human animal, comprising the steps of
(a) administering said candidate compound to said animal,
(b) obtaining a blood or tissue sample from said animal;
(c) qualitative and/or quantitative determination of the amount of protein according to SEQ ID NO: 1, of the nucleic acid according to SEQ ID NO: 2 or translational products thereof, in this sample;
(d) wherein the amount of protein or nucleic acid as determined in (c) being indicative of the candidate compound's ability of causing an allergy, inflammation, asthma or cancer or of glucocorticoid effects.


SEQUENCE LISTING

<110> GSF-Forschungszentrum für Gesundheit und Umwelt GmbH

<120> Use of a human chitinase-like protein in diagnosis and therapy

<130> P18186

<140> EP04027998.6
<141> 2004-11-25

<160> 4

<170> PatentIn version 3.3

<210> 1
<211> 393
<212> PRT
<213> Homo sapiens

<400> 1

<210> 2
<211> 1293
<212> DNA
<213> Homo sapiens

<400> 2
atggaatggg ctggggtcaa agactgggtg cctgggagct gaggcagcca ccgtttcagc 60

ctggccagcc ctctggaccc cgaggttgga ccctactgtg acacacctac catgcggaca 120

ctcttcaacc tcctctggct tgccctggcc tgcagccctg ttcacactac cctgtcaaag 180

tcagatgcca aaaaagccgc ctcaaagacg ctgctggaga agagtcagtt ttcagataag 240

ccggtgcaag accggggttt ggtggtgacg gacctcaaag ctgagagtgt ggttcttgag 300

catcgcagct actgctcggc aaaggcccgg gacagacact ttgctgggga tgtactgggc 360

tatgtcactc catggaacag ccatggctac gatgtcacca aggtctttgg gagcaagttc 420

acacagatct cacccgtctg gctgcagctg aagagacgtg gccgtgagat gtttgaggtc 480

acgggcctcc acgacgtgga ccaagggtgg atgcgagctg tcaggaagca tgccaagggc 540

ctgcacatag tgcctcggct cctgtttgag gactggactt acgatgattt ccggaacgtc 600

ttagacagtg aggatgagat agaggagctg agcaagaccg tggtccaggt ggcaaagaac 660

cagcatttcg atggcttcgt ggtggaggtc tggaaccagc tgctaagcca gaagcgcgtg 720

ggcctcatcc acatgctcac ccacttggcc gaggctctgc accaggcccg gctgctggcc 780

ctcctggtca tcccgcctgc catcaccccc gggaccgacc agctgggcat gttcacgcac 840

aaggagtttg agcagctggc ccccgtgctg gatggtttca gcctcatgac ctacgactac 900

tctacagcgc atcagcctgg ccctaatgca cccctgtcct gggttcgagc ctgcgtccag 960

gtcctggacc cgaagtccaa gtggcgaagc aaaatcctcc tggggctcaa cttctatggt 1020

atggactacg cgacctccaa ggatgcccgt gagcctgttg tcggggccag gtacatccag 1080

acactgaagg accacaggcc ccggatggtg tgggacagcc aggtctcaga gcacttcttc 1140

gagtacaaga agagccgcag tgggaggcac gtcgtcttct acccaaccct gaagtccctg 1200

caggtgcggc tggagctggc ccgggagctg ggcgttgggg tctctatctg ggagctgggc 1260

cagggcctgg actacttcta cgacctgctc tag 1293

<210>  3
<211>  21
<212>  DNA
<213>  Artificial

<220>
<223>  Primer

<400>  3
acgcacaagg agtttgagca g                                                21


<210>  4
<211>  20
<212>  PRT
<213>  Artificial

<220>
<223>  Primer

<400>  4

Gly Gly Ala Thr Thr Thr Thr Gly Cys Thr Thr Cys Gly Cys Cys Ala
1               5                   10                  15


Cys Thr Thr Gly
            20